# EUROPÄISCHE PATENTSCHRIFT

(11) **EP 1 771 157 B1**
(45) Veröffentlichungstag und Bekanntmachung des Hinweises auf die Patenterteilung: **12.03.2008**
(21) Anmeldenummer: 05758133.2
(22) Anmeldetag: 08.07.2005
(51) Int. Cl.: A61K 9/16, A61K 9/20, A61K 9/48, A61K 9/00, A61K 31/506, A61K 31/427, A61K 31/55

(54) **MULTIPARTIKULÄRE ARZNEIFORM FÜR WENIG LÖSLICHE WIRKSTOFFE SOWIE EIN VERFAHREN ZUR HERSTELLUNG DER ARZNEIFORM**
MULTIPARTICLE PHARMACEUTICAL DOSAGE FORM FOR LOW-SOLUBLE ACTIVE SUBSTANCES AND METHOD FOR PRODUCING SAID PHARMACEUTICAL DOSAGE FORM
FORME GALENIQUE MULTIPARTICULAIRE POUR PRINCIPES ACTIFS FAIBLEMENT SOLUBLES, ET PROCEDE DE PRODUCTION DE LA FORME GALENIQUE

(30) Priorität: 27.07.2004 DE 102004036437
(43) Veröffentlichungstag der Anmeldung: 11.04.2007
(73) Patentinhaber: Evonik Röhm GmbH, 64293 Darmstadt (DE)
(72) Erfinder: LIZIO, Rosario, 64380 Rossdorf (DE); PETEREIT, Hans-Ulrich, 64291 Darmstadt (DE); LANGGUTH, Peter, 63599 Biebergemünd (DE); KNÖLL, Marcus, 79539 Lörrach (DE)
(86) Internationale Anmeldenummer: PCT/EP2005/007427
(87) Internationale Veröffentlichungsnummer: WO 2006/010453

(56) Entgegenhaltungen:
- EP-A- 0 514 008
- WO-A-03/007913
- DE-A1- 10 332 160
- US-A- 5 938 990

## Beschreibung

Die Erfindung betrifft eine multipartikuläre Arzneiform, für wenig lösliche Wirkstoffe, die keine Peptide oder Proteine einschließlich deren Derivate oder Konjugate sind, sowie ein Verfahren zur Herstellung der Arzneiform.

### Stand der Technik

WO 02/03955 beschreibt bioadhaesive, microsphaerisch formulierte Arzneiformen zur sublingualen Wirkstoffapplikation. Die Microsphaeren haben einen mittleren Durchmesser von weniger als 50 µm und enthalten den Wirkstoff in nicht kristalliner Form in einer Micromatrix, eingebettet in ein bioadhaesives Polymer. Das bioadhaesive Polymer kann unter anderem eine Cellulose, ein Chitosan oder ein Acrylcopolymer sein.

WO 02/051390 beschreibt Formulierungen mit lipophiler Matrix und hohem Anteil (> 70 %) an Monoglyceriden, die eine effizientere Freisetzung des Wirkstoffes erlauben.

WO 02/64148 beschreibt Formulierungen enthaltend ein Mucopolysaccharid und ein Verfahren zu ihrer Herstellung. Dabei wird ein Mucopolysaccharid, z. B. Heparin, zusammen mit einem Adsorptionsverstärker, z. B. einem Chitosan, formuliert und anschließend mit einem darmsaftlöslichen Überzug ausgestattet, so daß der Wirkstoff in den mittleren oder unteren Abschnitten des Dünndarms freigesetzt werden kann. Als darmsaftlösliche Überzüge kommen z. B. anionische Acrylcopolymere vom Typ EUDRAGIT®L, S, L100-55 in Betracht. Die Formulierungen können Kapseln, Tabletten und Granulate umfassen.

WO 02/43767 beschreibt orale pharmazeutische Zusammensetzungen für physiologisch aktive Peptid-Wirkstoffe, enthaltend den Wirkstoff, der an einen Zellmembran Überträger gekoppelt ist, ein den pH-Wert absenkendes Mittel und/oder einen Protease Inhibitor sowie ein säurestabiles Transport Vehikel, welches die pharmazeutische Zusammensetzung auf dem Weg durch den Magen des Patienten schützt und einen Kontakt mit den im Magen gegenwärtigen Proteasen verhindert. Bei dem Transport Vehikel kann es sich um Kapseln handeln, die mit säureresistenten Überzügen aus EUDRAGIT® L30 D-55 überzogen sind.

WO 03/007913 beschreibt orale multipartikuläre Arzneiformen, die den Wirkstoff in Form einer Vielzahl sogenannter "Patches" enthalten. Ein "Patch" ist ein diskusartiges Objekt aus biokompatiblen Material mit einem Durchmesser von 500 µm bis 5 mm und einer Höhe von 100 bis 1000 µm. Das Patch besteht aus zwei Schichten bzw. Seiten, einer für Wasser oder Körperflüssigkeiten nur gering permeablen Seite, z. B. aus Ethylcellulose, und einer zweiten Seite, die den Wirkstoff, z. B. ein Peptid oder Protein, enthält, der in Mischung mit mucoadhaesiven Polymeren, z. B. Chitosan, CMC, Polyacrylsäure oder Pektin, vorliegen kann. Die Patches können zu einer Tablette verpresst werden oder auch in eine Kapsel gefüllt werden, die zusätzlich mit einem darmsaftlöslichen Überzug ausgestattet wird. Die Wirkstoffpräparationen können auch zusätzlich mit sogenannten Enhancem, wie Fettsäuren, fettalkoholen, Estern, oberflächenaktiven Substanzen und Proteaseinhibitoren kombiniert werden. Am Wirkort, z. B. im einem bestimmten Darmabschnitt, löst sich die Kapsel auf und gibt die "Patches" frei. Die freigesetzten "Patches" können mit ihrer mucoadhaesiven Seite an der Darmucosa anhaften und dort den Wirkstoff verzögert und auf die Darmucosa gerichtet abgeben. Die zweite, nur gering permeable Seite der "Patches" soll dem Wirkstoff einen gewissen Schutz gegenüber chemischer oder enzymatischer Inaktivierung von der Seite des Darmlumens her bieten und auch verhindern, daß der Wirkstoff nach dieser Seite hin entweicht.

EP0514008 offenbart eine feste Zusammensetzung bestehend aus Matrixpartikeln mit einem Schmelzbereich von 30-120 °C. Sie bestehen aus einem Viskositätserhöher (Acrylsäurepolymer), Polyglycerolfettsäureester oder einem Lipid, einem Wirkstoff (kein Peptid, kein Protein) und einem Überzug. Solche Zusammensetzungen heften sich an die Schleimhaut des Verdauungstraktes an und setzen den Wirkstoff über eine längere Zeitperiode frei.

### Aufgabe und Lösung

Die WO 03/007913 bietet eine beachtliche und anzuerkennende Lösung zur Bereitstellung von oralen Arzneiformen, insbesondere für Wirkstoffe, die im Darmlumen freigesetzt werden und dort zur Wirkung gelangen sollen. Ein Nachteil dieser Lösung besteht unter anderem in dem aufwendigen Aufbau und der Herstellung der zweischichtigen "Patch"-Strukturen. Ungünstig erscheint insbesondere aber die Bereitstellung der Arzneiform als Kapsel mit einem magensaftresistenten, darmsaftlöslichen Überzug. Bei einer Größe von deutlich über 2,5 mm ergibt sich hier eine ungenügende therapeutische Reproduzierbarkeit. Die Passagezeit der Kapsel durch den Magen kann stark variieren In jedem Fall ist mit einem verzögerten Wirkungseintritt zu rechnen. Außerdem kann sich die Kapsel schon nach einem teilweisen Auflösen des Überzugs ihrerseits schnell oder langsam auflösen. Beide Prinzipien, Überzug und Kapsel, überlagern sich hier in ungünstiger Weise, so daß mit einer insgesamt unkontrollierten Freisetzung der Patches" zu rechnen ist. Die Kapsel kann in einem Zustand, wo sie zumindest bereits teilweise den Darmsäften zugänglich ist, je nach aktuellem Darminhalt oder Darmperistaltik intakt bleiben oder auch mechanisch weitgehend zerstört werden. Es kann einerseits zu einer schlagartigen Freisetzung großer Mengen an "Patches" kommen oder andererseits auch zu einer ungewünscht verzögerten Freisetzung, je nach Zerfall oder mechanischer Belastung des zunächst überzogenen Kapselgebildes. Eine insgesamt besser zu kontrollierende Wirkstoffabgabe wäre daher wünschenswert.

Ein besonderes Problem ergibt sich bei den wenig löslichen Wirkstoffen im Sinne der DAB 10. Im Sinne der vorliegenden Erfindung sind dies Wirkstoffe, die eine Löslichkeit in Wasser nach DAB 10 von mindestens 30 Volumenteilen Wasser für einen Gewichtsteil Wirkstoff aufweisen. Die vorliegende Erfindung schließt Wirkstoffe, die Peptide oder Proteine sind, einschließlich deren Derivate oder Konjugate, ausdrücklich aus, da diese Gegenstand einer weiteren Erfindung sind. Wenig oder noch schlechter in Wasser lösliche Wirkstoffe bereiten Probleme bei der Formulierung der Arzneiform, vor allem aber weisen diese Wirkstoffe eine schlechte Bioverfügbarkeit auf. Aus diesem Grund werden viele solcher Wirkstoffe nur als parenterale Formulierungen weiterentwickelt, z.B. als Implantate oder Injektionen mit deutlichem Nachteil der Patienten-Compliance oder werden, trotz der bewiesenen pharmakologischen Wirkung, nicht als marktreife Formulierung weiterentwickelt. Wenn eine per-orale Darreichungsform dieser Wirkstoffe mit bekannten galenischen Ansätzen entwickelt wird, können grosse Teile des in der Arzneiform enthaltenen Wirkstoffs nicht vom Körper aufgenommen werden. Die verbleibende Bioverfügbarkeit hängt stark von den Umständen des Einzelfalls, z. B. den Umgebungsbedingungen und vermutlich auch vom aufnehmenden Organismus selbst ab und kann daher grossen Schwankungen unterliegen. Eine therapeutisch sichere Applikation ist deshalb in vielen Fällen nicht möglich. Es besteht somit ein Bedarf an Arzneiformen, mit denen wenig lösliche Wirkstoffe besser verabreicht werden können.

Es wurde als eine der Aufgaben der Erfindung gesehen, eine Arzneiform bereitzustellen, die sich zur gezielten und effektiven Freisetzung von wenig löslichen Wirkstoffen, ausgenommen Peptide oder Proteine, einschließlich deren Derivate oder Konjugate, eignet. Die Arzneiform soll eine hohe Dosiersicherheit bieten und sich nach schneller Magenpassage gut im Darmlumen verteilen. Der enthaltene Wirkstoff soll dabei weitgehend gegenüber physikalischer, chemischer oder enzymatischer Inaktivierung geschützt sein und am definierten Wirkort so freizusetzen sein, daß ein hoher Anteil des Wirkstoffs von Körper aufgenommen werden kann. Der Freisetzungsort soll je nach therapeutischem Ziel variabel und zuverlässig einstellbar sein.

Die Aufgabe wird gelöst durch eine

Orale multipartikuläre Arzneiform, in Form eines Behältnisses, das im pH-Bereich des Magens zerfällt, enthaltend eine Vielzahl von Pellets, Partikeln, Granulaten oder Agglomeraten mit einem mittleren Durchmesser im Bereich von 50 bis 2500µm, die im wesentlichen aufgebaut sind aus
a) einer inneren Matrix-Schicht, enthaltend einen Wirkstoff, der kein Peptid oder ein Protein einschließlich deren Derivate oder Konjugate ist, eine lipophile Matrix, die einen Schmelzpunkt oberhalb von 37 °C aufweist, und ein Polymer mit mucoadhaesiver Wirkung,
b) einem äußeren verfilmten Überzug, bestehend im wesentlichen aus einem anionischen Polymeren oder Copolymeren, das optional mit pharmazeutisch üblichen Hilfsstoffen formuliert sein kann,
dadurch gekennzeichnet, daß
der Wirkstoff eine Löslichkeit in Wasser nach DAB 10 von mindestens 30 Volumenteilen Wasser für einen Gewichtsteil Wirkstoff aufweist und in die lipophile Matrix eingebettet ist und die wirkstoffhaltige lipophile Matrix in eine Matrix aus dem Polymeren mit mucoadhaesiver Wirkung eingebettet ist.

### Ausführung der Erfindung

Die Erfindung betrifft eine Orale multipartikuläre Arzneiform, in form eines Behältnisses, insbesondere in form einer Tablette, Minitablette, in Kapseln verfüllter Pellets, Sachets oder Trockensäfte, das sich im pH-Bereich des Magens auflöst, enthaltend eine Vielzahl von Pellets, Partikeln, Granulaten, (z. B. auch Extrudaten, Co-Präzipitaten) oder Agglomeraten mit einem mittleren Durchmesser im Bereich von 50 bis 2500, bevorzugt von 100 bis 1000 µm, besonders bevorzugt von 200 bis 800 µm, die im wesentlichen aufgebaut sind aus

Einer inneren Matrix-Schicht a) enthält einen Wirkstoff, der kein Peptid oder ein Protein einschließlich deren Derivate oder Konjugate ist, eine lipophile Matrix, die einen Schmelzpunkt oberhalb von 37 °C aufweist, und ein Polymer mit mucoadhaesiver Wirkung. Optional bzw. in der Regel kann die innere Matrix-Schicht in der lipophilen Matrix oder zur Formulierung des Polymers mit mucoadhaesiver Wirkung weitere pharmazeutisch übliche Hilfsstoffe enthalten. Dem Fachmann sind solche Formulierungshilfsmittel bekannt.

Einem äußerer verfilmten Überzug b), besteht im wesentlichen aus einem anionischen Polymeren oder Copolymeren, das wiederum optional mit pharmazeutisch üblichen Hilfsstoffen, z. B. Trennmiteln und/oder Weichmachern formuliert sein kann. Die Formulierung schließt ein, das auch mehrere anionische Polymere oder Copolymere in Mischung vorliegen können.

Der Wirkstoff weist eine Löslichkeit in Wasser nach DAB 10 von mindestens 30 Volumenteilen Wasser für einen Gewichtsteil Wirkstoff auf und ist in die lipophile Matrix eingebettet. Die wirkstoffhaltige lipophile Matrix ist wiederum in eine Matrix aus dem Polymeren mit mucoadhaesiver Wirkung eingebettet.

Die multipartikuläre Arzneiform ist so formuliert, daß die enthaltenen Pellets, Partikel, Granulate, einschließlich Extrudaten, Co-Präzipitaten oder Agglomeraten im pH-Bereich des Magens freigesetzt werden. Das-Behältniss, das die Pellets, Partikel, Granulate, einschließlich Extrudate, Co-Präzipitate oder Agglomeraten enthält und im pH-Bereich des Magens zerfällt, kann z. B. eine Kapsel, eine Gelatinekapsel, eine Tablette, z. B. eine verpresste Tablette, eine Trockensaftformulierung oder ein Sachet sein.

Der äußere Überzug ist durch die Wahl des anionischen Polymeren bzw. Copolymeren bzw. seiner Formulierung mit Hilfsstoffen und seiner Schichtdicke so eingestellt, daß sich dieser in pH-Bereichen von 4,0 bis 8,0, bevorzugt von 5,5 bis 7,8, besonders bevorzugt 5,8 bis 7,5 im Darm innerhalb 10 bis 60, bevorzugt von 20 bis 40 min auflöst, so daß die mucoadhaesive Matrix-Schicht frei wird, an die Darmmucosa binden und dort die lipophile wirkstoffhaltige Matrix in Kontakt mit dem Enterozyten bringen kann. Somit kann der Wirkstoff mit oder ohne Komponenten der lipophile matrix resorbiert werden. Die lipophile Matrix ist so gewählt, dass sich der Wirkstoff und die Substanz oder die Substanzen, die die lipophile Matrix bilden, sich in ihrer Löslichkeit in Wasser nach DAB 10 und nicht mehr als +/- 50 % unterscheiden und/oder in ihrem Verteilungskoeffizienten nach Anhang V zu RL 67/548/EWG, A.8, nach nicht mehr als +/- 60 % unterscheiden und/oder in ihrem HLB-Wert gemessen nach Marszall nicht mehr als +/- 80 % unterscheiden.

Das Polymere bzw. Copolymere mit mucoadhaesiver Wirkung ist so gewählt, daß es in einem Bereich +/- 0,5, bevorzugt +/- 0,3 pH-Einheiten bezogen auf den pH-Wert, bei dem sich der äußere Überzug aufzulösen beginnt, eine mucoadhaesive Wirkung von mindestens ηb= 150 bis 1000, bevorzugt 200 bis 900 mPa·s und eine Wasseraufnahme von 10 bis 750, bevorzugt 10 bis 250, besonders bevorzugt 10 bis 160 % in 15 min aufweist und der Wirkstoffanteil der Matrixschicht maximal 40, insbesondere 0,001 bis 15 oder 0,05 bis 5-Gew.-% des Gehaltes am Polymeren mit mucoadhaesiver Wirkung beträgt.

### Die innere Matrix Schicht

Die innere Matrix-Schicht fungiert als Wirkstoffträger. Weiterhin hat die innere Matrix-Schicht die Funktion, den Wirkstoff in der lipophilen Matrix zu lösen oder zumindest zu binden, nach Möglichkeit seine Bioverfügbarkeit zu erhöhen. Mittels der die lipophile Matrix umgebenden Matrix aus dem mucoadhaesiven Polymeren können Bestandteile der inneren Schicht an die Darmmucosa binden. Der Wirkstoff kann von dort aus in den Organismus gelangen kann. Die innere Matrix Schicht hat weiterhin die Funktion den Wirkstoff vor physikalischer, chemischer oder enzymatischer Inaktivierung zu schützen.

Die innere Matrix-Schicht besteht aus zwei Matrix-Komponenten:
a) Eine lipophile Matrix-Komponente, in der der Wirkstoff aufgelöst, dispergiert, emulgiert oder einfach gemischt ist und
b) Eine mucoadhesive Matrix-Komponente, in der die lipophile Matrix eingebettet ist.

Die innere lipophile Matrix-Komponente fungiert als Wirkstoffträger. Weiterhin hat die innere Matrix-Schicht die Funktion, den Wirkstoff in der lipophilen Matrix zu lösen oder zumindest zu binden, nach Möglichkeit seine Bioverfügbarkeit zu erhöhen. Zusätzlich hat die innere Matrix-Schicht die Funktion, den Wirkstoff mittels der enthaltenen Lipiden und/oder amphiphile Substanzen gegen die physikalische, chemische oder enzymatische Degradation zu schützen, die Penetration und die Resorption durch das Darm-Epithelium (Enterozyten) zu fordern, so dass dieser von dort aus in den Organismus gelangen kann.

Die weitere Matrix-Schicht oder Kern aus mucoadhesiven Polymeren, in denen die lipophile Matrix eingebettet ist, fungiert als zweiter Träger. Weiterhin hat diese Schicht oder der Kern die Funktion, die lipophile Matrix mittels des enthaltenen mucoadhaesiven Polymeren an die Darmmucosa zu binden, so daß dieser von dort aus, durch die Mucus-Schichte an die Oberflächen der Enterozyten gelangen kann und somit den direkten Kontakt der lipophilen Matrix mit den Enterozyten erlauben kann. Die mucoadhaesive Matrix Schicht oder Kern hat weiterhin die Funktion, die lipophile Matrix zusätzlich vor physikalischer, chemischer oder enzymatischer Spaltung/Inaktivierung zu schützen

### Wirkstoffe/Wirkstoffformulienrungen

### Wirkstoffe

Die im Sinne der Erfindung eingesetzten Wirkstoffe sind insbesondere dazu bestimmt, am oder im menschlichen oder tierischen Körper Anwendung zu finden, um
1. Krankheiten, Leiden, Körperschäden oder krankhafte Beschwerden zu heilen, zu lindem, zu verhüten oder zu erkennen.
2. die Beschaffenheit, den Zustand oder die Funktionen des Körpers oder seelische Zustände erkennen lassen.
3. vom menschlichen oder tierischen Körper erzeugte Wirkstoffe oder Körperflüssigkeiten zu ersetzen.
4. Krankheitserreger, Parasiten oder körperfremde Stoffe abzuwehren, zu beseitigen oder unschädlich zu machen oder
5. die Beschaffenheit, den Zustand oder die Funktionen des Körpers oder seelische Zustände zu beeinflussen.

Die Wirkstoffe, die der keine Peptid oder Proteine einschließlich deren Derivate oder Konjugate sind, können als freie Säuren oder Basen eingesetzt werden. Als Gegenionen können beispielsweise physiologisch Basen oder Säuren, verträgliche Erdalkali- oder Alkalimetalle oder Amine sowie beispielsweise Acetat, Adipat, Ascorbat, Alginat, Benzoat, Benzoolsulfonat, Bromid, Carbonat, Carboxymethylcellulose (freie Säure), Citrat, Chlorid, Dubutylphosphat, Dihydrogencitrat, Dioctylphosphat, Dihexadecylphosphat, Fumarat, Gluconat, Glucuronat, Glutamat, Hydrogencarbonat, Hydrogentartrat, Hydrochlorid, Hydrogencitrat, Jodid, Lactat, alpha-Liponat, Malat, Maleat,Malonat, Pamoat, Palmitat, Phosphat, Salicylat, Stearat, Succinat, sulphat, Tartrat, Tannate, Oleat, Octylphosphat eingesetzt werden.

Der Wirkstoffanteil der lipophilen Matrix kann z. B. maximal 90, insbesondere 1 bis 60 oder 5 bis 50 Gew.% betragen. Der Gehalt der wirkstoffhaltigen lipophilen Matrix an der inneren Matrix-Schicht a) kann bevorzugt 5 bis 60, besonders bevorzugt 10 bis 50 Gew.-% betragen.
In Abhängigkeit der physiko-chemischen Eigenschaften des Wirkstoffs, wie z. B. Partitionskoeffizient Wasser in Öl oder isoelektrischer Punkt etc., kann die innere Schicht a) zusätzlich einen Efflux-Pumpen-Inhibitor, z. B. Ketokonazol oder Polyethylen-660-12-Hydroxy-Stearat (Solutol® HS15), enthalten.

Die innere Schicht a) kann zusätzlich einen Penetrationsförder.er insbesondere Weichmacher wie beispielsweise Triethylcitrat, Acetyltrietylcitrat, Diethylsebacat, Dibutylsebacat, Polymere wie Carbomer, Chitosan, -Chitosan-Cystein, Natrium-Carboxymethyhellulose, N-Trimethyliertes Chitosan, Polycarbophil-Cysteine, langkettige Fettsäuren, ihre Ester (beispielsweise Mono und Diglyceride) und ihre Salze wie Laurinsäure, Laurinsulfonsäure, Palmitinsäure, Caprylsäure, Caprinsäure, Ölsäure, Acylcarnitine, Chelatbildner wie EDTA, Salicylate, Cyclodextrine, Polyacrylsäuren, Gallensäuren wie Cholsäure, Cholyltaurin, Cholylsarcosin, Chenodeoxycholsäure und ihre Salze wie Na-Cholat, Na-Glykocholat, Na-Taurocholat, Na-Taurodihydrofusidat, Na-Glykodihydrofusidat, Tenside und Emulgatoren wie Polysorbat 80 (Tween 80), Polyoxyethyliertes Kastoröl (Cremophor EL), Polyoxyethylene-polyoxypropylene glycol (Pluronic® F68), das Toxin Zonula Occludens Toxin (ZOT) sowie Vitamine wie Vitamin E (Tocopherol) oder Vitamin B12 enthalten.

Die innere Schicht a) kann zusätzlich Enzym-Inhibitoren enthalten, z. B. Lipase-inhibitoren, Esterase-inhibitoren und/oder Glycolase-inhibitoren.

### Polymere mit mucoadhaesiver Wirkung

Die innere Matrix-Schicht a) enthält weiterhin Polymere mit mucoadhaesiver Wirkung, die die wirkstoffhaltige lipophile Matrix umhüllen bzw. einbetten. Geeignete Polymere mit mucoadhaesiver Wirkung sind insbesondere ein Chitosan (Chitosan und Derivate, Chitosane), (Meth)acrylatcopolymere, bestehend aus 20-45 Gew.-% Methylmethacrylat und 55 bis 80 Gew.-% Methacrylsäure, Cellulosen, insbesondere Methylcellulosen, wie Na-Carboxymethylcellulose (z. B. Blanose® oder Methocel®).

Das Polymer mit mucoadhaesiver Wirkung ist so gewählt, daß es in einem Bereich +/- 0,5, bevorzugt +/- 0,3 pH-Einheiten bezogen auf den pH-Wert, bei dem sich der äußere Überzug aufzulösen beginnt, eine Wasseraufnahme von 10 bis 750, bevorzugt 10 bis 250, besonders bevorzugt 10 bis 160 % in 15 min aufweist.

### Messung der mucoadhäsiven Eigenschaften

Eine geeignete Messmethode zur Charakterisierung mucoadhäsiver Eigenschaften ist in *Hassan und Gallo* (1990) enthalten (s. *Hassan E.E. und Gallo J.M. "A Simple Rheological Method for the in Vitro Assessment of Mucin-Polymer Bioadhaesive Bond Strength" Pharma Res.* 7 (5), 491 (1990)). Die Methode beruht auf der Annahme, daß die Viscosität (η, dynamische Viscosität bzw. Viscositätskoeffizient) einer Mischung von Polymeren mit Mucin verschieden ist, von der Summe der Viskositäten der Einzelkomponenten. Es gilt der Zusammenhang
η_{Mischung Polymer mit Mucin} = η_{Mucin} + η_{Polymer} + η_{b}, wobei η_{b} für die Differenz steht. Je höher η_{b}, desto höher sind die mucoadhaesiven Eigenschaften. Die einzelnen Komponenten werden zunächst mit einem Rotationsviscosimeter auf ihre Viscosität hin vermessen. Eingesetzt werden eine 0,5 %-ige (w/w) wässrige Lösung des mucoadhaesiven Polymeren und eine 15 %-ige Lösung von Mucin aus Schweinemagen. Zur Bestimmung der mucoadhaesiven Eigenschaften η_{b} werden Mucin und Polymer allein und in ihrer Mischung in den angegebenen Konzentrationen gemessen.

Das Polymer mit mucoadhaesiver Wirkung ist so gewählt, daß es in einem Bereich +/- 0,5, bevorzugt +/- 0,3 pH-Einheiten bezogen auf den pH-Wert, bei dem sich der äußere Überzug aufzulösen beginnt, eine mucoadhaesive Wirkung gemessen als Viskosität η_{b} von 150 bis 1000, bevorzugt 150 bis 600, mPa·s aufweist.

### Hydratisierung und Wasseraufnahme

Die Hydratisierung von Polymeren beruht auf der Affinität des Polymers Wasser aufzunehmen. Polymere schwellen durch diese Wasseraufnahme an. Dies liegt an einem Ungleichgewicht zwischen dem chemischen Potential des Wassers im Polymer und dem Wasser im umgebenden Medium. Das Wasser wird aufgrund des osmotischen Druckes des Polymers solange aufgenommen bis sich ein Gleichgewicht zwischen innerer und äußerer Phase eingestellt hat. Das Polymer ist dann zu 100% hydratisiert. Für Polymere mit niedrigem mittleren Molekulargewicht liegt dann eine Lösung vor. Für Polymere mit höherem Molekulargewicht oder vernetzten Polymeren entsteht ein Gel. Die Wasseraufnahme bis zur Gleichgewichtseinstellung kann z. B. bis zum 10 fachen des eigenen Gewichtes betragen entsprechend 1000% des Polymergewichtes.

### Messung der prozentualen Wasseraufnahme

Die Messung der prozentualen Wasseraufnahme ist dem Fachmann geläufig. Eine geeignete Methode ist z. B. im Lehrbuch der pharmazeutischen Technologie/Rudolf Voigt, Basel: Verlag Chemie, 5. völlig überarbeitete Auflage, 1984, S. 151, 7.7.6 unter "Aufsaugvermögen" beschrieben. Die Methode bedient sich der sogenannten Enslin-Apparatur, bei der eine Glasfilternutsche über einen Schlauch mit einer graduierte Pipette verbunden ist. Die Pipette ist genau horizontal so montiert, daß sie auf gleicher Höhe mit der Glasfritte liegt. Eine Wasseraufnahme von 100 % wird im vorliegenden Fall als eine Wasseraufnahme von 1 ml Wasser pro 1 g Polymeren mit mucoadhaesiver Wirkung in 15 min definiert.

Durch die vergleichsweise schnelle Wasseraufnahme bzw. Hydratisierung und den hohen Hydratisierungsgrad wird zu dem Zeitpunkt, ab dem sich der äußere Überzug aufzulösen beginnt, ein rascher Schutz des Wirkstoffs und eine unmittelbare Bindung an die Darmmucosa gewährleistet. Die Bindung des Wirkstoffs in der mucoadhaesiven Matrix soll nur gering sein, so daß dieser unmittelbar von der Darmmucosa in den Organismus übergehen kann.

### Steuerung des pH-Wertes in der Matrix, enthaltend das Polymer mit mucoadhaesiver Wirkung

Die mucoadhaesive Wirkung ist bei vielen mucoadhaesiven Polymeren pHabhängig. Der pH-Wert in der Matrix kann gezielt durch den Zusatz einer Säure, einer Base oder eines Puffersystems gesteuert werden. Die innere Matrix kann beispielsweise als Polymer mit mucoadhaesiver Wirkung ein Chitosan enthalten, das zusammen mit einem Acetat-Puffersystem eingesetzt wird. Der Acetat/Na-Acetat-Puffer, z. B. auf pH 5,0 bis 5,5 eingestellt, kann sich als Zusatzstoff in der Matrix befinden oder auf einem Kern auf dem die Matrix aufgetragen wird aufgebracht. Auf diese Weise kann Chitosan einschließlich dessen Derivate auch in Kombination mit verfilmten Überzügen eingesetzt werden, die sich bei höheren pH-Werten, z. B. pH 6,0 bis 8,0, aufzulösen beginnen. Trotz des hohen Umgebungs-pH-Wertes, bleibt der niedrige pH-Wert in der Mikroumgebung der Matrix erhalten. Man kann so die mucoadhaesiven Eigenschaften des Polymers in einem pH-Bereich nutzen, in dem es ansonsten nicht oder nicht in diesem Maße mucoadhaesiv wirken würde. Dies hat den Vorteil, daß man einen gewissen Schutz gegenüber Enzymen erzielen kann, deren pH-Optimum in höheren pH-Bereichen liegt. Das gleiche Prinzip kann auch in umgekehrter Weise angewendet werden, indem man den-pH-Wert der Matrix mittels des Zusatzes einer Base erhöht und mit einem verfilmten Überzug kombiniert, der sich bei niedrigeren pH-Werten auflöst.

### Beispiele zur Auswahl geeigneter mucoadhaesiver Polymere

Die Auswahl geeigneter mucoadhaesiver Polymere basiert auf ihren mucoadhaesiven Eigenschaften und ihrer Wasseraufnahmefähigkeit. Die Polymere sollen im jeweiligen pH-Bereich eine mucoadhaesive Wirkung von mindestens η_{b} = 150 bis 1000 mPa·s und eine Wasseraufnahme von 10 bis 750 % in 15 min aufweisen. Die folgende Tabelle gibt eine beispielhafte Auflistung.

Chitosan ist z. B. geeignet für die Anwendung in einem Umgebungs-pH-Bereich von pH 5,5 (Duodenum) oder bei einem anderen Umgebungs-pH-Bereich (Ileum oder Colon), sofern der Matrix-pH-Bereich, z. B. mit Hilfe eines Puffersystems, auf den Bereich um pH 5,5 eingestellt wurde.

Das in der Tabelle aufgelistete (Meth)acrylatcopolymer ist besser für einen Ph-Bereich von pH 7,2 als für einen pH-Berich um pH 5,5 geeignet.

Na-Alginat ist für den pH-Bereich um pH 5,5 geeignet, jedoch nicht für pH 7,2.

Na-Carboxymethylcellulose und vernetzte Polyacrylsäure sind über einen weiten pH-Bereich von 5,5 bis 7,2 geeignet.

| Mucoadhesives Polymer | Mucoadhaesive Wirkung η_{b} [mPa·s] bei pH 5,5 | Mucoadhaesive Wirkung η_{b} [mPa·s] bei pH 7,2 | H₂O- Aufnahme [% in 15 min] bei pH 5,5 | H₂O- Aufnahme [% in 13 min] bei pH 6,0 | H₂O-Aufnahme [% in 15 min] bei pH 7,2 |
|---|---|---|---|---|---|
| Chitosan | 220 | 0 | 140 | 320 | 320 |
| (Meth)acrylatcopolymer* | 150 | 480 | 170 | 50 | 125 |
| Na-Alginat | 580 | 0 | 40 | 50 | 50 |
| Na-Carboxymethyl-cellulose | 300 | 250 | 55 | 50 | 50 |
| Polyacrylsäure vernetzt | 350 | 340 | 50 | 25 | 25 |

| | | | | | |
|---|---|---|---|---|---|
| *= (Meth)acrylatcopolymer aus 30 Gew.-% Methylmethacrylat und 70 Gew.-% Methacrylsäure | | | | | |

### Der äußere Überzug b) aus anionischen (Meth)acrylatcopolymeren

Der äußere verfilmte Überzug b) aus anionischen Polymeren oder copolymeren dient als magensaftresistenter Überzug zum Schutz der inneren Schicht a) vor den Magensäften. Weiterhin fungiert der äußere Überzug zum Schutz des Wirkstoffs vor einer möglichen physikalischen, chemischen oder eventuell sogar enzymatischen Inaktivierung bis zu dem Zeitpunkt, an dem sich der Überzug einen Darmabschnitt (Duodenum, Jejunum, Ileum oder Colon) erreicht, an dem er sich aufzulösen beginnt. Der äußere Überzug dient dabei insbesondere dem sogenannten "gastrointestinalen Targeting", d. h. der gezielten Freisetzung der inneren Matrixschicht, an den durch ihren dort herrschenden pH-Wert bestimmten Darmabschnitten. Damit es nicht zu einer Behinderung der Abgabe der inneren Schicht a) kommt, soll das (Meth)acrylatcopolymere des äußeren Überzugs möglichst keine oder nur geringe Wechselwirkungen mit dem Wirkstoff oder dem mucoadhaesiven Polymeren der inneren Schicht aufweisen.

Geeignete anionische Polymere bzw. Copolymere sind Celluloseglycolat (Duodcell®), Celluloseacetatphtalat (CAP, Cellulosi acetas, PhEur, Celluloseacetate-phtalate, NF, Aquateric®), Celluloseacetatsuccinat (CAS), Celluloseacetattrimeliat (CAT), Hydroxypropylmethylcellulosephtalat (HPMCP, HP50, HP55), Hydroxypropylmethylcellulose acetatsuccinat (HPMCAS -LF, -MF, -HF), Polyvinylacetatphtalat (PVAP, Sureteric®), Vinylacetat-Vinylpyrolidon-Copolymer (PVAc, Kollidon® VA64), Vinylacetat:Crotonsäure-Copolymer 9:1 (VAC:CRA, Kollicoat® VAC) und oder Shelllack. Die genannten Polymere bzw. Copolymere lassen sich vielfach in durchaus befriedigender Weise so formulieren, daß eine pH-spezifische Auflösung erreicht werden kann.

Besonders bevorzugt besteht der äußere verfilmte Überzug im wesentlichen aus (Meth)acrylat-Copolymeren mit einem Gehalt an Monomeren mit anionischen Gruppen von 5 bis 60 Gew.-%, die optional mit pharmazeutisch üblichen Hilfsstoffen, insbesondere Weichmachern formuliert sein können. Gegenüber den eingangs genannten Polymeren bieten die genannten anionischen (Meth)acrylatcopolymere im Rahmen der Erfindung die Möglichkeit in vielen Fällen eine noch genauere und reproduzierbarere pH-spezifische Einstellung des Auflöse-pH-wertes einzustellen. Auch die Handhabung und Applikation wird in der Regel als weniger aufwendig angesehen.

Das (Meth)acrylat-Copolymere für den äußeren Überzug besteht bevorzugt zu 40 bis 95, bevorzugt zu 45 bis 90, insbesondere zu 30 bis Gew.% aus radikalisch polymerisierten C₁- bis C₄-Alkylestern der Acryl- oder der Methacrylsäure und kann 5 bis 60, bevorzugt 8 bis 40, insbesondere 20 bis 35 Gew.-% (Meth)acrylat-Monomere mit einer anionischen Gruppe enthalten.

In der Regel addieren sich die genannten Anteile zu 100 Gew.-%. Es können jedoch zusätzlich, ohne daß dies zu einer Beeinträchtigung oder Veränderung der wesentlichen Eigenschaften führt, geringe Mengen im Bereich von 0 bis 10, z. B. 1 bis 5 Gew.-% weiterer vinylisch copolymerisierbarer Monomere, wie z. B. Hydroxyethylmethacrylat oder Hydroxyethylacrylat enthalten sein.

C₁- bis C₄-Alkylestern der Acryl- oder Methacrylsäure sind insbesondere Methylmethacrylat, Ethylmethacrylat, Butylmethacrylat, Methylacrylat, Ethylacrylat und Butylacrylat.

Ein (Meth)acrylat-Monomer mit einer anionischen Gruppe kann z. B. Acrylsäure, bevorzugt jedoch Methacrylsäure sein.

Weiterhin geeignet sind anionische (Meth)acrylat Copolymere aus 40 bis 60, Gew.-% Methacrylsäure und 60 bis 40 Gew.-% Methylmethacrylat oder 60 bis 40 Gew.-% Ethylacrylat (Typen EUDRAGIT® L oder EUDRAGIT® L100-55).

EUDRAGIT® L ist ein Copolymer aus 50 Gew.% Methylmethacrylat und 50 Gew.% Methacrylsäure. EUDRAGIT® L 30D ist eine Dispersion enthaltend 30 Gew.-% EUDRAGIT® L. Dieses (Meth)acrylatcopolymer ist besonders geeignet zur Auflösung in pH-Bereichen um pH 6,0 bis 6,5 (Jejunum).

EUDRAGIT® L100-55 ist ein Copolymer aus 50 Gew.-% Ethylacrylat und 50 Gew.-% Methacrylsäure. EUDRAGIT® L 30-55 ist eine Dispersion enthaltend 30 Gew.-% EUDRAGIT® L 100-55. Dieses (Meth)acrylatcopolymer ist besonders geeignet zur Auflösung in pH-Bereichen um pH 5,5 bis 6,0 (Duodenum)

Ebenso geeignet sind anionische (Meth)acrylat Copolymere aus 20 bis 40 Gew.% Methacrylsäure und 80 bis 60 Gew.-% Methylmethacrylat (Typ EUDRAGIT® S). Dieses (Meth)acrylatcopolymer ist besonders geeignet zur Auflösung in pH-Bereichen um pH 6,5 bis 7,0 (Jejunum bzw. Ileum)

Besonders gut geeignet sind (Meth)acrylat Copolymere, bestehend aus 10 bis 30 Gew.-%, Methylmethacrylat, 50 bis 70 Gew.-% Methylacrylat und 5 bis 15 Gew.-% Methacrylsäure.

EUDRAGIT® FS ist ein Copolymer aus 25 Gew.-%, Methylmethacrylat, 65 Gew.% Methylacrylat und 10 Gew.-% Methacrylsäure. EUDRAGIT® FS 30 D ist eine Dispersion enthaltend 30 Gew.-% EUDRAGIT® FS. Dieses (Meth)acrylatcopolymer ist besonders geeignet zur Auflösung in pH-Bereichen um pH 7,0 bis 7,8 (Ileum bzw. Colon)

Weiterhin geeignet ist ein Copolymer, welches sich aus
20 bis 34 Gew.-% Methacrylsäure und/oder Acrylsäure,
20 bis 69 Gew.-% Methylacrylat und
0 bis 40 Gew.% Ethylacrylat und/oder gegebenenfalls
0 bis 10 Gew.-% weiteren vinylisch copolymerisierbarer Monomeren
zusammensetzt, mit der Maßgabe, daß die Glastemperatur des Copolymers nach ISO 11357-2, Punkt 3.3.3, höchstens 60 °C beträgt. Dieses (Meth)acrylatcopolymer ist wegen seiner guten Reißdehungseigenschaften insbesondere zum Verpressen von Pellets zu Tabletten geeignet.

Weiterhin geeignet sind Copolymere aus
20 bis 33 Gew.-% Methacrylsäure und/oder Acrylsäure,
5 bis 30 Gew.-% Methylacrylat und
20 bis 40 Gew.-% Ethylacrylat und
größer 10 bis 30 Gew.-% Butylmethacrylat und gegebenenfalls
0 bis 10 Gew.% weiteren vinylisch copolymerisierbarer Monomeren,
wobei sich die Anteile der Monomeren zu 100 Gew.-% addieren,
zusammensetzt, mit der Maßgabe, daß die Glastemperatur des Copolymers (glass transition temperature) nach ISO 11357-2, Punkt 3.3.3 (midpoint temperature *T*_{mg}), 55 bis 70 °C beträgt. Copolymere dieses Typs sind wegen seiner guten mechanischen Eigenschaften insbesondere-zum Verpressen von Pellets zu Tabletten geeignet.

Das oben genannte Copolymer setzt sich insbesondere zusammen aus radikalisch polymerisierten Einheiten von
20 bis 33, bevorzugt 25 bis 32, besonders bevorzugt 28 bis 31 Gew.-% Methacrylsäure oder Acrylsäure, bevorzugt ist Methacrylsäure,
5 bis 30, bevorzugt 10 bis 28, besonders bevorzugt 15 bis 25 Gew.-% Methylacrylat,
20 bis 40, bevorzugt 25 bis 35, besonders bevorzugt 18 bis 22 Gew.% Ethylacrylat, sowie
größer 10 bis 30, bevorzugt 15 bis 25, besonders bevorzugt 18 bis 22 Gew.-% Butylmethacrylat
zusammen, wobei die Monomerzusammensetzung so gewählt wird, daß die Glastemperatur des Copolymers 55 bis 70 °C, bevorzugt 59 bis 66, besonders bevorzugt 60 bis 65 °C beträgt.

Zur Einstellung spezieller Freisetzungsprofile bzw. Freisetzungsorte können auch Mischungen der genannten Copolymere zum Einsatz kommen.

Unter Glastemperatur wird hier insbesondere die midpoint temperature *T*_{mg} nach ISO 11357-2, Punkt 3.3.3, verstanden. Die Messung erfolgt ohne Weichmacherzusatz, bei Restmonomergehalten (REMO) von weniger als 100 ppm, bei einer Aufheizrate von 10 °C/min und unter Stickstoffatmosphäre.

Das Copolymer besteht bevorzugt in wesentlichen bis ausschließlich, zu 90, 95 oder 99 bis 100 Gew.%, aus den Monomeren Methacrylsäure, Methylacrylat, Ethylacrylat und Butylmethacrylat in den oben angegebenen Mengenbereichen.

Es können jedoch zusätzlich, ohne daß dies zu einer Beeinträchtigung der wesentlichen Eigenschaften führen muß, geringe Mengen im Bereich von 0-bis 10, z. B. 1 bis 5 Gew.% weiterer vinylisch copolymerisierbarer Monomere, wie z. B. Methylmethacrylat, Butylacrylat, Hydroxyethylmethacrylat, Vinylpyrrolidon, Vinylmalonsäure, Styrol, Vinylalkohol, Vinylacetat und/oder deren Derivate enthalten sein.

Die genannten Copolymere sind größtenteils handelsüblich oder können in an sich bekannter Weise durch radikalische Substanz-, Lösungs-, Perl- oder Emulsionspolymerisation erhalten werden. Sie müssen vor der Verarbeitung durch geeignete Mahl-, Trocken- oder Sprühprozesse in den erfindungsgemäßen Teilchengrößenbereich gebracht werden.
Dies kann durch einfaches Brechen extrudierter und abgekühlter Granulatstränge oder Heißabschlag erfolgen.

Insbesondere bei Mischung mit weiteren Pulvern oder Flüssigkeiten kann der Einsatz von Pulvern vorteilhaft sein. Geeignete Gerätschaften zur Herstellung der Pulver sind dem Fachmann geläufig, z. B. Luftstrahlmühlen, Stiftmühlen, Fächermühlen. Gegebenenfalls können entsprechende Siebungsschritte einbezogen werden. Eine geeignete Mühle für industrielle Großmengen ist zum Beispiel eine Gegenstrahlmühle (Multi Nr. 4200), die mit ca. 6 bar Überdruck betrieben wird.

### Copolymerherstellung

Die genannten (Meth)acrylatcopolymere sind durch radikalische Polymerisation der Monomeren erhältlich (siehe z. B. EP 0 704 207 A2 und EP 0 704 208 A2). Die Copolymere sind in an sich bekannter Weise durch radikalische Emulsionspolymerisation in wäßriger Phase in Gegenwart von vorzugsweise anionischen Emulgatoren herstellbar, beispielsweise nach dem in DE-C 2 135 073 beschriebenen Verfahren.

### Organische Lösung

Die genannten (Meth)acrylatcopolymere können in Form einer organischen Lösung, z. B. in einer Konzentration von 10 bis 30 Gew.-%, bereitgestellt werden. Als Lösungsmittel können z. B. Aceton, Isopropanol oder Ethanol oder Mischung daraus verwendet werden, die gegebenenfalls Wasseranteile bis etwa 10 Gew.-% enthalten können . Bevorzugt sind jedoch wäßrige Dispersionen.

### Dispersionen

Die genannten (Meth)acrylatcopolymere können als Emulsionspolymerisate vorzugsweise in Form einer 10- bis 50-gew.-prozentigen, insbesondere 20 bis 40-prozentigen wäßrigen Dispersion erzeugt und angewendet werden. Als Handelsform ist ein Feststoffgehalt von 30 Gew.-% bevorzugt. Für die Verarbeitung ist eine teilweise Neutralisation der Methacrylsäure-Einheiten entbehrlich; sie ist jedoch, beispielsweise in einem Umfang bis zu 5 oder 10 Mol-% möglich, wenn eine Stabilisierung oder Verdickung der Überzugsmitteldispersion erwünscht sein sollte. Der Gewichtsmittelwert der Latex-Teilchengröße beträgt in der Regel 40 bis 100 nm, vorzugsweise-50 bis 70 nm, was eine verarbeitungstechnisch günstige Viskosität unter 1000 mPa· s gewährleistet.

Bei höheren Neutralisationsgrades z. B. 10 bis 50 Mol.% oder vollständiger Neutralisation ist es möglich, das Copolymer in einen gelösten Zustand zu überführen.

Um eine Lösung des anionischen Copolymers herzustellen ist in der Regel eine teilweise oder vollständige Neutralisation der Säuregruppen nötig. Das anionische Copolymer kann z. B. nach und nach in einer Endkonzentration von 1 bis 40 Gew.-% in Wasser eingerührt werden und dabei teilweise oder vollständig neutralisiert werden durch Zugabe einer basischen Substanz wie z. B. NaOH, KOH; Ammoniumhydroxyd oder organische Basen wie z. B. Triethanolamin. Es ist auch möglich ein Pulver des Copolymeren einzusetzen, dem bereits bei seiner Herstellung zum Zweck der (Teil)neutralisation eine Base z. B. NaOH zugesetzt wurde, so daß das Pulver ein bereits (teil)neutralisiertes Polymer ist. Der pH-Wert der Lösung liegt in der Regel über 4, z. B. im Bereich von 4 bis ca. 7.

Die Dispersion kann z. B. auch in an sich bekannter Weise sprühgetrocknet oder gefriergetrocknet werden und im Form eines redispergierbaren Pulvers bereitgestellt werden (siehe z. B. EP-A 0 262 326). Alternative Verfahren sind die Gefriertrocknung oder Coagulation uns Abquetschen des Wassers in einem Extruder mit anschließender Granulation (siehe z. B. EP-A 0 683 028).

Überraschenderweise wurde gefunden, daß Copolymer-Dispersionen aus sprüh- oder gefriergetrockneten und redispergierten Pulvern eine erhöhte Scherstabilität aufweisen. Dies ist insbesondere beim Sprühauftrag von Vorteil. Dieser Vorteil tritt insbesondere verstärkt hervor wenn das in der Dispersion enthaltene Copolymer zu 2 bis 10 Mol-% in teilneutralisierter Form vorliegt (bezogen auf die im Copolymer enthaltenen Säuregruppen). Bevorzugt ist zu diesem Zweck die Teilneutalisation mittels Zugabe von NaOH. Bevorzugt ist ein anionischer Emulgator in Menge von 0,1 bis 2 Gew.-% enthalten. Besonders bevorzugt ist Natiumlaurylsulfat als Emulgator.

### Schichtdicken

Die Schichtdicke des äußeren Überzuges liegt bevorzugt im Bereich von 20 bis 200, bevorzugt von 50 bis 120 *µ*m.

### Vorteilhafte Wirkungen der Erfindung

Die erfindungsgemäße Arzneiform eignet sich zur gezielten und effektiven Freisetzung von wenig oder noch schlechter in Wasser lösliche Wirkstoffe. Die Arzneiform weist eine hohe Dosiersicherheit und verteilt sich gut im Darmlumen. Der enthaltene wenig oder noch schlechter in Wasser lösliche Wirkstoffe wird dabei weitgehend gegenüber physikalischer oder enzymatischer Inaktivierung geschützt und kann am definierten Wirkort so freigesetzt werden, daß ein hoher Anteil des Wirkstoffs von Körper aufgenommen werden kann. Die Arzneiform kommt daher mit weniger Wirkstoff aus, da nur wenig Wirkstoff verloren geht. Die Gefahr von Nebenwirkungen wird durch die gezielte Abgabe insgesamt verringert. Der Wirkort kann je nach therapeutischem Ziel variabel eingestellt werden. Der Zeitpunkt der Wirkstoffaufnahme kann somit besser gesteuert werden. Da es sich um eine orale Arzneiform handelt hat diese eine insgesamt bessere Akzeptanz der Patienten ("patient compliance") im Vergleich zu anderen Applikationsformen. Eine Vielzahl von wenig oder noch schlechter in Wasser lösliche Wirkstoffe wird dadurch erstmals der peroralen Anwendung zugänglich und haben somit weniger Applikationsrisiken wie insbesondere bei parenteralen Applikationen. Auch die Kosten der Applikation können gering gehalten werden, da kein Fachpersonal für die Applikation notwendig ist.

Eine beschleunigte Freigabe bei gleichzeitiger Steigerung der Bioverfügbarkeit kann erreicht werden aus Matrixsystemen, in denen der Anteil des Polymeren mit mucoadhaesiver Wirkung in Gew.% 2-fach, bevorzugt 10 bis 200-fach höher ist als der Wirkstoffanteil.

### Lipophile Matrix

Ein besonderer Aspekt der Erfindung ergibt sich wenn der Wirkstoff in eine lipophile Matrix eingebettet ist, die einen Schmelzpunkt (bestimmt nach Differential Scanning Calorimetrie, DSC) oberhalb von 37°C, bevorzugt oberhalb von 40 °C, besonders bevorzugt oberhalb von 45 °C aufweist und wirkstoffhaltige lipophile Matrix in die Matrix aus dem Polymeren mit mucoadhaesiver Wirkung eingebettet ist. Die Formulierung in der lipophilen Matrix zielt darauf ab, die Löslichkeit bzw. die Bioverfügbarkeit des Wirkstoffs, bevorzugt von wenig oder schwerlöslichen Wirkstoffen (im Sinne der DAB 10) zu verbessern.

Unter einer lipophilen Matrix im Sinne der Erfindung versteht man eine Substanz oder eine Mischung von Substanzen, in der oder denen der Wirkstoff gelöst, suspendiert oder emulgiert werden kann. Die Substanz oder die Substanzen der lipophilen Matrix sind verschieden von den üblichen pharmazeutischen Hilfsstoffen und dem Polymer mit mucoadhaesiver Wirkung. Die Substanz oder die Substanzen der lipophilen Matrix haben bevorzugt einen hydrophoben oder auch amphiphilen Charakter. Man könnte die lipophile Matrix auch als amphiphile Matrix oder als lipoide Matrix bezeichnen.

Die lipophile Matrix kann aus einer einzelnen Substanz, z. B. einem Lipid, oder einer Mischung von Substanzen, z. B einer Mischung von Lipiden, bestehen. Im Falle von Mischungen errechnen sich die im folgenden beschriebenen Eigenschaften für Wasserlöslichkeiten nach DAB 10, Partitionskoeffizienten und/oder HLB-Werte jeweils aus dem arithmetrischen Mittel aus den Gewichtsteilen und den Werten der Substanzen der Mischung. Die eingesetzten Substanzen dürfen nicht toxisch sein.

Der Wirkstoff und die Substanz oder die Substanzen, die die lipophile Matrix bilden, unterscheiden sich bevorzugt in ihrer Löslichkeit in Wasser nach DAB 10 und nicht mehr als +/- 50 %, bevorzugt nicht mehr als +/- 25 % unterscheiden und/oder in ihrem Verteilungskoeffizienten nach Anhang V zu RL 67/548/EWG, A.8 nicht mehr als +/- 60 %, bevorzugt nicht mehr als +/- 30 % unterscheiden und/oder in ihrem HLB-Wert, sofern ein den Substanzen ein HLB Wert zugeordnet werden kann, gemessen nach Marszall nicht mehr als +/- 80 %, bevorzugt nicht mehr als +/- 40 % unterscheiden. Je höher die Übereinstimmung des Wirkstoffs mit der lipophilen Matrix in zumindest einer, bevorzugt zweier oder allen drei der genannten Eigenschaften, desto mehr ist die Löslichkeit und die Bioverfügbarkeit des Wirkstoffs in der Arzneiform begünstigt.

### Löslichkeit in Wasser

Die Löslichkeit in Wasser für den Wirkstoff und die Substanz oder die Substanzen, die die lipophile Matrix bilden, kann nach DAB 10 (Deutsches Arzneibuch, 10. Ausgabe mit 3. Nachtrag 1994, Deutscher Apothekerverlag, Stuttgart und Govi Verlag, Frankfurt a. M., 2. Nachtrag (1993), IV Allgemeine Vorschriften, S. 5 - 6, "Löslichkeit und Lösungsmittel"; s. a. Ph. Eur. 4.07, 2004), definiert werden. Die Definition der Löslichkeit erfolgt über die Anzahl der Volumenteile Lösungsmittel für 1 Gewichtsteil Substanz, bzw. Arzneistoff. Die Definition "wenig löslich" umfasst Substanzen, die über 30 bis 100 Volumenteile Lösungsmittel für 1 Gewichtsteil Substanz, bzw. Arzneistoff erfordern, die Definition "schwer löslich" umfasst Substanzen, die über 100 bis 1000 Volumenteile Lösungsmittel für 1 Gewichtsteil Substanz, bzw. Arzneistoff erfordern.

### Verteilungskoeffizienten

Die Verteilungskoeffizienten für den Wirkstoff und die Substanz oder die Substanzen, die die lipophile Matrix bilden, können nach Anhang V zu RL 67/548/EWG, A.8 "verteilungskoeffizient" bestimmt werden.

### HLB-Wert

Der HLB-Wert ist ein 1950 von Griffin eingeführtes Maß der Hydrophilie bzw. Lipophilie von nichtionischen Tensiden. Er läßt sich experimentell durch die Phenol-Titrationsmethode nach Marszall bestimmen; vgl. "Parfümerie, Kosmetik", Band 60, 1979, S. 444 - 448; weitere Literaturhinweise in Römpp, Chemie-Lexikon, 8.Aufl. 1983, S.1750. Siehe weiterhin z. B. US 4 795 643 (Seth)). Ein HLB-Wert (Hydrophile/Lipophile-Balance) läßt sich nur bei nichtionischen Substanzen exakt bestimmen. Bei anionischen Substanzen kann dieser Wert rechnerisch ermittelt werden, liegt jedoch praktisch immer über oder weit über 14.

HLB-Werte für den Wirkstoff und die Substanz oder die Substanzen, die die lipophile Matrix bilden, können in den meisten Fällen nach Marszall bestimmt werden, aus Tabellen pharmazeutischer oder chemischer Nachschlagewerke oder Lehrbücher entnommen werden oder nach bei ionischen Substanzen rechnerisch ermittelt werden.

### Wirkstoffe in der lipophilen Matrix

Bevorzugt enthält die Arzneiform in der lipophilen Matrix einen Wirkstoff, der eine Löslichkeit in Wasser nach DAB 10 von mindestens 30, insbesondere über 30 bis 100 oder über 100 bis 1000 Volumenteilen Wasser für einen Gewichtsteil Wirkstoff aufweist. Der bevorzugte Wirkstoff ist demnach wenig oder sogar schwer löslich nach der Definition der DAB 10.

Der in der lipophilen Matrix fomulierte Wirkstoff kann aus z. B. aus der Gruppe der BCS-Klasse II und IV (Bio-Pharmaceutical-Classification-System nach Prof. Amidon) ausgewählt werden. Die Wirkstoffe der BCS-Klassen sind dem Fachmann bekannt. Der in der lipophilen Matrix fomulierte Wirkstoff kann z. B. aus der Gruppe der Antiandrogene, Antidepressiva, Antidiabetika, Antirheumatika, Glucocorticoide, Cytostatika, Migränemittel, Neuroleptika, Antibiotika, Östrogene, Vitamine, Psychopharmaca, ACE-Hemmer, β-Blocker, Ca-Kanalblocker, Diuretika, Herzglykoside, Antiepileptika, Diuretika / Antiglaukom, Urikostatika, H₂-Rezeptorenblocker und Virostatika ausgewählt werden.

Der in der lipophilen Matrix fomulierte Wirkstoff kann z. B. Bicalutamid, Anastrozol, Glimiprid, Nilutamid, Bromocriptin, Ketotifen, Letrozol, Naratriptan, Ganciclovir, Orlistat, Mesoprosztol, Granistron, Pioglitazone, Lamivudine, Rosiglitazon, Zidovudin, Enalapril, Atenolol, Nadolol, Felodipin, Bepridil, Furosemid, Digoxin, Digitoxin, Carbamazepin, Acetazolamid, Allopurinol; Cimetidin, Ranitidin, Oxcarbazepin sein.

### Lipophile Matrix/Polymere mit mucoadhaesiver Wirkung

In einer bevorzugten Ausführungsform werden mögliche Interaktionen der lipophilen Matrix mit dem Polymer mit mucoadhaesiver Wirkung berücksichtigt. Um nicht kontrollierbare Wechselwirkungen zu vermeiden, soll die Substanz oder die Substanzen, die die lipophile Matrix bilden, und das Polymere mit mucoadhaesiver Wirkung bevorzugt entweder die gleichen ionische Eigenschaften aufweisen, d.h. beide sollen übereinstimmend entweder zumindest überwiegend kationischen oder übereinstimmend anionischen Charakter haben. Im Falle, daß die Substanzen mit entgegengesetzten ionischer Eigenschaften ausgewählt werden, soll das Polymere mit mucoadhaesiver Wirkung bevorzugt zu mindestens 50, besonders bevorzugt zu 100 % in neutralisierter Form vorliegen. Die Neutralisation kann durch Zusatz von Säure oder Base in bekannter Weise erfolgen.

### Substanz oder Substanzen für den Aufbau der lipophilen Matrix

Bevorzugt besteht die lipophile Matrix zu 80 bis 100, bevorzugt zu 90 bis 100, besonders bevorzugt zu 100 Gew.-% aus einer Substanz oder einer Mischung von Substanzen mit einem (gemittelten) HLB-Wert von 0 bis 15, bevorzugt 2 bis 10 besteht. Die lipophile Matrix kann 0 bis 20, bevorzugt 0 bis 10 Gew.-% an pharmazeutisch üblichen Hilfsstoffen, insbesondere Stabilisatoren, Verdickungsmittel oder Adsorbentien, enthalten. Besonders bevorzugt sind keine pharmazeutisch üblichen Hilfsstoffe enthalten.

Die Substanz oder die Substanzen, die die lipophile Matrix bilden, können z. B. zu der Gruppe der Öle, Fette, Mono-, Di- oder Triglyceride, Fettsäuren, Fettalkohole, insbesondere C₅- bis C₂₀-Fettsäure und/oder einen C₅- bis C₂₀-Alkohol einschließlich deren Salze, Ether-, Ester oder Amid-Derivaten, Phospholipide, Lecithine, Emulgatoren, Lipoide, lipidlösliches Vitamine oder Tenside gehören.

Die lipophile Matrix kann z. B. eine der folgenden Lipidpräparationen enthalten: (Imwitor 308) Glycerylmonocaprylate mit einem Monoesteranteil >80 %, (Imwitor 312) Glycerylmonolaurate mit enem Monoesteranteil > 90 %, (Imwitor 491) Glycerolmonosterate (C₁₆ + C₁₈) mit einem Monoesteranteil >90 %, (Imwitor 900 P) Glycerolmonosterat mit einem Monoesteranteil von 40 - 55% und einem C₁₈-Gehalt von 40 - 60%, (Imwitor 900 K) Glycerolmonosterat, mit einem Monoesteranteil von 40 - 55 % und einem C₁₈-Gehalt von -60 -80 %, (Imwitor 742) Mittelkettige C₈ und C₁₀Glyceride mit einem Monoesteranteil von 45 - 55 %, (Imwitor 928) Partielle Glyceride gesättigter pflanzlicher C₁₀-C₁₈Fettsäuren mit einem Hauptanteil an C₁₂, und mit einem Monoesteranteil von 34-36%, C₈ and C₁₀-Glyceride, NaCaprylat oder NaCapriat.

### Die lipophile Matrix kann z. B. eine der folgenden Lipidpräparationen enthalten:

Die lipophile Matrix kann aus einer einzigen Komponente oder aus einer Mischung von Fetten oder Lipiden wie Mono-, Di-, Triglyceride von gesättigten und ungesättigten Fettsäuren bestehen. Insbesondere Glycerin-Stearinsäureester, Glycerin-Palmitinsäureester, Glycerin-Myristinsäureester, Glycerin-Palmitinsäure-Stearinsäureester, Glycerin-Laurinsäureester Glycerin-Caprylsäureester, Glycerin-Ölsäureester, Beispiele für diese Ester sind Imwitor® - 308, - 312, - 491, 742, - 900, - 928, - 988, sowie Gelucire® 44/14, - 50/13, Geleol, Compritol E ATO, Dynasan 114, Softisan, Witepsol, Dynacet 212, Kokosfett.
Wachse wie beispielsweise Kanaubawachs, Bienenwachs, Wollwachs Glycerinbehensäureester.
Öle wie beispielsweise Rizinusöl, Sesamöl, Sonnenblumenöl,
Baumwollsamenöl, Maisöl, Mandelöl, Erdnussöl, Olivenöl, Kokosnussöl, Karottenöl, Weizenkeimöl, Walnussöl.
**Neutralöle** wie Isopropylmyristat, -palmitat, -stearat, mittelkettige Triglyceride (Miglyol^{®}).
**Fettalkohole** wie beispielsweise Stearylalkohol, Laurylalkohol, Cetylalkohol, Myristinalkohol, Glycerinformal.
**Fettsäureamide** wie beispielsweise Stearinsäureamid, Palmitinsäureamid, Laurinsäureamid.
**Kurzkettige aliphatische und aromatische Carbonsäureester wie** beispielsweise Dibutylphthalat, Diethylsebacat, Dibutylsebacat, Tributylcitrat, Acetyltributylcitrat, Glycerintriacetat.
**Aliphatische langkettige Carbonsäuren** wie beispielsweise Stearinsäure, Palmitinsäure, Laurinsäure, Myristinsäure, Ölsäure, Caprylsäure, Linolsäure, Linolensäure, Arachidonsäure. Sowie beispielsweise deren Na-, Al- und Mg-Salze.
**Aliphatische kurzkettige und mittelkettige Carbonsäuren**, wie beispielweise Valeriansäure, Isovaleriansäure, Capronsäure, Önanthsäure, Caprylsäure, Pelargonsäure, Caprinsäure.
Sowie beispielsweise deren **Na-, Al- und Mg-Salze.**
**W/O Emulgatoren** wie beispielsweise Cholesterol, Glycerinmonostearat, Ethylenglycolmonostearat, Sorbitanmonooleat (Span® 80), -palmitat.(Span® 40), -laurat (Span® 20), -stearat (Span® 60), Sorbitantrioleat (Span® 85)" Sorbitantristearat (Span® 65), Sorbitansesquioleate (Arlacel® 83), Ca-, Al, Mg-Stearat, Polyoxethylensorbitantristearat (Tween® 65), Polyoxethylensorbitantrioleat (Tween® 85).
**Nichtionische OIW Emulgatoren** wie beispielsweise Macrogolstearat 400 - (Chremophor® A), Macrogollaurylether,- Polyethylenglykol-20-Sorbitanmonolaurat, -stearat, -palmitat, -oleat, Macrogol-1500-glycerintriricinoleat, Macrogolglycerinhydroxy-stearat (Cremophor® RH), Macrogol-1000-glycerinmono-laurat, -stearat, -oleat, Saccharosemonostearat. Polysorbat 60 (Tween® 60), Polyoxyethylenmonostearat (Myrj 49), Polysorbat 80 (Tween® 80), Polysorbat 40 (Tween® 40), Polysorbat 20 (Tween® 20), Poloxamer 407 (Lutrol®F 127), Poloxamer 188 (Lutrol® F 68), Polyoxyethlylenrizinoleat (Cremophor® EL), Polyoxyethylen-5-stearylstearat,
**lonische O/W Emulgatoren** wie beispielsweise Cetylstearylsulfat (Lanette®E), Na-Laurylsulfat (Texapon® Z), Na-Glycocholat, Hederagenin,
**Amphiphile Emulgatoren** wie beispielsweise Phospholipiden, Lecithine, Ei-Phosphatidylcholin (Eilecithin), Soja-Phosphatidylcholin (Sojalecithin), Betain, Sulfobetaine, Ceramide (Sphingomyelin),
**Vitamine** wie beispielsweise Retinol (Vitamin A), Cholecalciferol (Vitamin D), alpha-Tocopherol und alpha-Tocopherolacetat (Vitamin E) und deren derivaten, Phyllochinon (Vitamin K),
Galaktolipide wie beispielsweise Monogalaktosyl-diacylglycerin, Digalaktosyldiacylglycerin, Trigalaktosyl-diacylglycerin,
Aromatische Öle wie beispielsweise Anisöl, Citronellöl, Eukalyptusöl,
Fenchelöl, Kamillenöl, Kardamomenöl, Kiefernadelöl, Kümmelöl, Latschenöl, Lavendelöl, Minzöl, Muskatöl, Nelkenöl, Pfefferminzöl, Rosmarinöl, Salbeiöl, Terpene wie beispielsweise Menthol, Linalool, 1,4-Cineol, Pyrethrin, Bomeol, Eudesmol, Phytol, Manool, Azadirachtin, Nimbin,
bestehen, die spezifisch für die Einstellung der gewünschte Löslichkeit, Partitionskoeffizient (auch als Verteilungskoeffizient Öl/Wasser bekannt) oder HLB-Wert als einzelne oder als Mischung ausgewählt werden können.

Bevorzugt ist der Wirkstoff zu mindestens 10 %, besonders bevorzugt zu mindestens 20 %, insbesondere zu mindestens 50 % in der lipophilen Matrix löslich.

Der Gehalt der wirkstoffhaltigen lipophilen Matrix an der inneren Matrix-Schicht a) kann bevorzugt 5 bis 60, besonders bevorzugt 10 bis 50 Gew.-% betragen.

Bevorzugt enthält die lipophile Matrix zu mindestens 50 Gew.% Glycerinmonocaprylat, bis zu 10 Gew.-% Na-Cholat, bis zu 10 Gew.-% Tocopherol-Succinat, 1 bis 5 Gew.% eines Efflux-Pumpen-Inhibitors im Falle, daß der Wirkstoff ein Substrat der Pgp-Effluxpumpe ist, z. B. Solutol HS 15, ein Triglycerid, insbesondere Tristearat, wobei sich die Komponenten zu 100 % addieren. Diese lipophile Matrix kann direkt ins mucoadhaesive Polymer eingearbeitet werden oder in Wasser emulgiert in das mucoadhaesive Polymer eingearbeitet werden. Im letzteren Fall kann die Wasserphase eine schwache Säure, wie z. B. Citronensäure, beinhalten.

### Verfahren

### Herstellung von Prä-Pellets und Pellets

Die Pelletierung kann auf wirkstofffreie Kugeln (Nonpareilles) erfolgen oder es können kemfreie Pellets erzeugt werden.

Zunächst wird die innere Matrixschicht mit oder ohne Kern erzeugt. Man kann diese noch nicht überzogene, ausgerundete Schicht als Prä-Pellet bezeichnen.

### Ausführung ohne neutralen Kern

Zunächst wird der Wirkstoff in der lipophilen Matrix aufgelöst dispergiert oder gemischt. Dafür sind alle bekannten pharmazeutischen Verfahren anwendbar. Dann wird die lipophile Matrix in wässrigem Medium emulgiert oder dispergiert oder aufgelöst, je nach dem was die Lipophilizität und die chemischphysikalisch Eigenschaften der Matrix erlauben.

Als wässrigem Medium kann einfach Wasser gelten oder wässrige Zubereitungen von Säuren, schwachen Basen und Salzen. Zum Beispiel 15%iger Essigsäure-Lösung.
Die so entstandene Emulsion, Dispersion oder Lösung wird als Bindemittel für die Weiterbearbeitung der muco-adhaesiven Polymeren in Pellet-form benützt.

Die nun zusammengebrachten Inhaltsstoffe der inneren Matrix-Schicht a) können durch Verfahren wie Rotagglomeration, Ausfällen, Extrusion.

Granulierung oder Sprühverfahren, insbesondere Ultraschall-Wirbel-Sprühverfahren, zu noch nicht überzogenen Pellets (Prä-Pellets) von definierter Größe, z. B. 50 bis 1000µm, ausgerundet werden. Dies hat den Vorteil, daß das gesamte Pelletvolumen zur Wirkstoffbeladung zur Verfügung steht.

Im nächsten Abschnitt wird die Ausführung am Beispiel der Rotoragglomeration erläutert In einem Wirbelschichtgerät (z.B. GPCG1 von Glatt) mit Rotor-Ansatz wird das mucoadhesive Polymer-Pulver mit Mikrokristalline Cellulose-Pulver (MCC) vorgelegt. Der Anteil von MCC kann bis 50 % des gesamten Pulveranteils betragen. Luftstrom sowie Rotor-Ansatz werden gestartet und das Bindemittel gesprüht. Somit wird die wässrige Phase die Quellung des mucoadhesiven Polymers und die Einfeuchtung der MCC hervorrufen, die für die Bildung der Prä-Pellets durch die zentrifugale Kraft von Rotor-Ansatz notwendig ist. Der Anteil an MCC dient auch zur besseren Verformbarkeit der Masse und daher auch zur Steuerung der physikalische Eigenschaften der resultierenden Prä-Pellets (z.B. Dichte, Abriebfestigkeit, Form, etc.). Während des Bildungsprozesses verteilt sich der lipophile Anteil des Bindemittels auf den Oberflächen der Pulver-Partikeln und bleibt somit homogen in Form von Mikro-Domänen in den Pellets verteilt bzw. eingebettet.

### Ausführung mit neutralem Kern

In dieser Variante wird die lipophile und/oder amphiphile Lösung des Wirkstoffs in einem wässrigen Medium emulgiert, dispergiert oder aufgelöst, das bereits das mucoadhesive Polymer (als Dispersion oder Lösung) enthält.
Diese Emulsion, Dispersion oder Lösung wird dann auf einem neutralen Kern mittels bekannter Wirbelschichtgeräte aufgesprüht und als Schicht gebildet.
Um eine befriedigende Sprühbarkeit des Gemisches zu gewährleisten, ist es meist notwendig, ein Gemisch mit niedriger Viscosität zu formulieren. Zu diesem Zweck kann es günstig sein, das Polymere mit mucoadhaesiver Wirkung in vergleichsweise niedrigen Konzentrationen, z. B. von 1 bis höchstens 10, bevorzugt 2 bis 5 Gew.-% einzusetzen. Weiterhin kann der Zusatz eines Detergenz, z. B. Tween in Konzentrationen von 0,1 bis 20, bevorzugt 0,5 bis 10 Gew.% zur Herabsetzung der Oberflächenspannung vorteilhaft sein.

### Neben dem Wirkstoff können sie weitere pharmazeutische Hilfsstoffe enthalten:

Bindemittel, wie Zellulose und deren Derivate, Polyvinylpyrrolidon (PVP), Feuchthaltemittel, Zerfallsförderer, Gleitmittel, Sprengmittel, (Meth)acrylate, Stärke und deren Derivate, Zucker Solubilisatoren oder andere.

Entsprechende Auftragsverfahren sind z. B. aus Bauer, Lehmann, Osterwald, Rothgang, "Überzogene Arzneiformen" Wissenschaftliche Verlagsgesellschaft mbH Stuttgart, Kap 7, S.165 -196, bekannt.

Details sind dem Fachmann weiterhin aus Lehrbüchem bekannt. Siehe z. B.:
- Voigt, R. (1984): Lehrbuch der pharmazeutischen Technologie; Verlag Chemie Weinheim - Beerfield Beach/Florida - Basel.
- Sucker, H., Fuchs, P., Speiser, P. : Pharmazeutische Technologie, Georg Thieme Verlag Stuttgart (1991), insbesondere Kapitel 15 und 16, S. 626 -642.
- Gennaro, A.,R. (Editor), Remington's Pharmaceutical Sciences, Mack Publishing Co., Easton Pennsylvania (1985), Chapter 88, S. 1567- 1573.
- List, P. H. (1982): Arzneiformenlehre, Wissenschaftliche Verlagsgesellschaft mbH, Stuttgart.

Nach Herstellung der inneren Matrix-Kerne (bzw. der Prä-Pellets) werden diese wiederum bevorzugt im Sprühverfahren mit dem äußeren Überzug versehen, so daß man fertige Pellets erhält: Die Herstellung der Pellets erfolgt mittels Sprühauftrag aus organischer Lösung, oder bevorzugt aus wäßrigen Dispersionen. Für die Ausführung ist dabei entscheidend, daß gleichmäßige, porenfreie Überzüge entstehen.

### Topcoat

Die Pellets können zusätzlich mit pigmentierten Überzügen versehen werden, die jedoch nicht den Auflöse-pH-Wert beeinflussen dürfen. Geeignet sind z. B. Überzüge aus pigmentierter Hydroxypropylmethylcellulose oder anderen wasserlöslichen bzw. in Wasser schnell zerfallenden Polymeren.

### Pharmazeutisch übliche Hilfsstoffe

Den erfindungsgemäßen Formulierungen können bei der Herstellung übliche Hilfs- bzw. Zuschlagstoffe hinzugefügt werden. Grundsätzlich müssen natürlich alle eingesetzten Substanzen toxikologisch unbedenklich und insbesondere in Arzneimitteln ohne Risiko für Patienten zu verwenden sein.

Einsatzmengen und Verwendung der üblichen Zuschlagstoffe in Arzneimittelüberzügen oder Beschichtungen sind dem Fachmann geläufig. Übliche Zuschlagstoffe können z. B. Weichmacher, Trennmittel, Pigmente, Stabilisatoren, Antioxidantien, Porenbildner, Penetrationsförderer, Glanzmittel, Aromastoffe, Detergenzien, Schmierstoffe oder Geschmacksmittel sein. Sie dienen als Verarbeitungshilfsmittel und sollen ein sicheres und reproduzierbares Herstellungsverfahren sowie gute Langzeitlagerstabilität gewährleisten oder sie erreichen in der Arzneiform zusätzliche vorteilhafte Eigenschaften. Sie werden den Polymerzubereitungen vor der Verarbeitung zugesetzt und können die Permeabilität der Überzüge beeinflussen, was ggf. als zusätzlicher Steuerparameter genutzt werden kann.

### • Trennmittel:

Trennmittel besitzen in der Regel lipophile Eigenschaften und werden in der Regel den Sprühsuspensionen zugesetzt. Sie verhindern eine Agglomeration der Kerne während der Befilmung. Bevorzugt werden Talkum, Mg- oder Ca - Stearat, gemahlene Kieselsäure, Kaolin oder nicht ionische Emulgatoren mit einem HLB - Wert zwischen 3 und 8 eingesetzt. Übliche Einsatzmengen für Trennmittel in den erfindungsgemäßen Überzugs- und Bindemitteln liegen zwischen 0,5 bis 100 Gew.% bezogen auf das Copolymer.

### • Pigmente:

Mit dem Überzugsmittel unverträgliche Pigmente sind insbesondere solche Pigmente, die wenn sie der (Meth)acrylat-Copolymer-Dispersion direkt zugesetzt werden, z. B. durch Einrühren, in üblichen Anwendungsmengen von z.B. 20 bis 400 Gew.-% bezogen auf das Trockengewicht des (Meth)acrylat-Copolymeren zur Destabilisierung der Dispersion, Koagulation, zu Entmischungserscheinungen oder ähnlich unerwünschten Effekten führen. Weiterhin sind die zu verwendenden Pigmente natürlich nicht toxisch und für pharmazeutische Zwecke geeignet. Siehe dazu z. B. auch: Deutsche Forschungsgemeinschaft, *Farbstoffe für Lebensmittel,* Harald Boldt Verlag KG, Boppard (1978); Deutsche Lebensmittelrundschau 74, Nr. 4, S. 156 (1978); Arzneimittelfarbstoffverordnung AmFarbV vom 25:08.1980.

Mit dem Überzugsmittel unverträgliche Pigmente können z. B. Aluminiumoxidpigmente sein. Unverträgliche Pigmente sind z. B., Gelborange, Cochenillerotlack, Farbpigmente auf Basis von Aluminiumoxid bzw Azofarbstoffen, Sulfonsäurefarbstoffe, Gelborange S (E110, C.I. 15985, FD&C Yellow 6), Indigocarmin (E132, C.I. 73015, FD&C Blue 2), Tartrazin.(.E 102, C.I. 19140, FD&C Yellow 5), Ponceau 4R (E 125, C.I. 16255, FD&C Cochineal Red A), Chinolingleb (E 104, C.I. 47005, FD&C Yellow 10), Erythrosin (E127, C.I. 45430, FD&C Red 3), Azorubin (E 122, C.I. 14720, FD&C Carmoisine), Amaranth (E 123, C.I. 16185, FD&C Red 2), Brilliantsäuregrün (E 142, C.I. 44090, FD&C Green S).

Die angegebenen E-Nummem der Pigmente beziehen sich auf eine EU-Nummerierung. Siehe dazu auch "Deutsche Forschungsgemeinschaft, Farbstoffe für Lebensmittel, Harald Boldt Verlag KG, Boppard (1978); Deutsche Lebensmittelrundschau 74, Nr. 4, S. 156 (1978); Arzneimittelfarbstoffverordnung AmFarbV vom 25.08.1980. Die FD&C-Nummem beziehen sich auf die Zulassung in Food, Drugs und Cosmetics durch U.S. Food and Drug Administration (FDA) beschrieben in: U.S. Food and Drug Administration, Center for Food Safety and Applied Nutrition, Office of Cosmetics and Colors: Code of Federal Regulations - Title 21 Color Additive Regulations Part 82, Listing of Certified Provisionally Listed Colors and Specifications (CFR 21 Part 82).

### • Weichmacher

Weitere Zuschlagstoffe können auch Weichmacher sein. Übliche Mengen liegen zwischen 0 und 50, bevorzugt 2 bis 20, insbesondere 5 bis 10 Gew.-%.

Weichmacher können je nach Typ (lipophil oder hydrophil) und zugesetzter Menge die Funktionalität der Polymerschicht beeinflussen. Weichmacher erreichen durch physikalische Wechselwirkung mit dem Polymeren eine Absenkung der Glasübergangstemperatur und fördern in Abhängigkeit von der zugesetzten Menge die Verfilmung. Geeignete Stoffe haben in der Regel ein Molekulargewicht zwischen 100 und 20.000 und enthalten eine oder mehrere hydrophile Gruppen im Molekül, z. B. Hydroxyl-, Ester- oder Aminogruppen.

Beispiele geeigneter Weichmacher sind Citronensäurealkylester, Glycerinester, Phthalsäurealkylester, Sebacinsäurealkylester, Succroseester, Sorbitanester, Diethylsebacat, Dibutylsebacat und Polyethylenglykole 200 bis 12.000. Bevorzugte Weichmacher sind Triethylcitrat (TEC) und Acetyltriethylcitrat (ATEC). Weiterhin zu nennen sind in der Regel bei Raumtemperatur flüssige Ester wie Citrate, Phthalate, Sebacate oder Rizinusöl. Bevorzugt werden Zitronensäure- und Sebacinsäureester verwendet.

Die Zugabe der Weichmacher zur Formulierung kann in bekannter Weise, direkt, in wäßriger Lösung oder nach thermische Vorbehandlung der Mischung vorgenommen werden. Auch können Mischungen von Weichmachem eingesetzt werden

### -Herstellung multipartikulärer Arzneiformen

Die wirkstoffhaltigen, überzogenen Pellets können mittels pharmazeutisch üblicher Hilfsstoffe und in an sich bekannter Weise zu multipartikulären Arzneiformen, insbesondere zu pellethaltigen Tabletten, Minitabletten, Kapseln, Sachets oder Trockensäften verarbeitet werden, die so formuliert sind, daß die enthaltenen Pellets im pH-Bereich des Magens freigesetzt werden. Die Darstellung als multipartikuläre Arzneiform stellt eine hohe Dosiersicherheit bietet den Vorteil einer guten Verteilung der Pellets im Darmlumen. Die erfindungsgemäße multipartikuläre Arzneiform kann zudem auch verschiedene Pellettypen mit unterschiedlichen Wirkstoffen und/oder unterschiedlichem Pellet-Aufbau enthalten.

### Verpreßte Tabletten

Die Herstellung von multipartikulären Arzneiformen durch Verpressen eines pharmazeutisch üblichen Bindemittels mit wirkstoffhaltigen Partikeln ist z. B. Beckert et al. (1996), "Compression of enteric-coated pellets to disintegrating tablets,,, International Journal of Pharmaceutics 143, S. 13 - 23, und in WO 96/01624 beschrieben.

Filmüberzüge auf wirkstoffhaltige Pellets werden üblicherweise in Wirbelschichtgeräten aufgebracht. Rezepturbeispiele sind in dieser Anmeldung erwähnt. Filmbildner werden üblicherweise mit Weichmachem und Trennmitteln nach einem geeigneten Verfahren gemischt. Hierbei können die Filmbildner als Lösung oder Suspension vorliegen. Die Hilfsstoffe für die Filmbildung können ebenfalls gelöst oder suspendiert sein. Organische oder wässrige Löse- oder Dispergiermittel können verwendet werden. Zur Stabilisierung der Dispersion können zusätzlich Stabilisatoren verwendet werden (Beispiel: Tween 80 oder andere geeignete Emulgatoren bzw. Stabilisatoren).

Beispiele für Trennmittel sind Glycerolmonostearat oder andere geeignete Fettsäurederivate, Kieselsäurederivate oder Talkum. Beispiele für Weichmacher sind Propylenglykol, Phthalate, Polyethylenglykole, Sebacate oder citrate, sowie andere in der Literatur erwähnte Substanzen.

Zwischen wirkstoffhaltiger und darmlöslicher Copolymer-Schicht kann eine trennende Schicht aufgebracht sein, die der Trennung von Wirkstoff und Überzugsmaterial zum Zwecke der Verhinderung von Interaktionen dient. Diese Schicht kann aus inerten Filmbildnem (z.B. HPMC, HPC oder (Meth)acrylsäure-Copolymeren) oder z.B. Talkum oder einer anderen geeigneten pharmazeutischen Substanzen bestehen. Ebenso können Kombinationen aus Filmbildnern und Talkum oder ähnlichen Stoffen verwendet werden.

Es ist auch möglich, eine Trennschicht aus teilweise bzw. vollneutralisierten (Meth)acrylatcopolymer-Dispersionen aufzubringen.

Zwischen der inneren Schicht a) und der äußeren verfilmten Überzugsschicht b) kann eine Trennschicht aufgebracht werden. Die Trennschicht kann auch aus dem gleichen oder einem anderen mucoadhaesiven Polymer wie in der unterliegenden Schicht bestehen. Auf diese Weise kann eventuellen Interaktionen oder Unverträglichkeiten des Wirkstoffs oder des mucoadhaesiven Polymers mit der filmbildenden (Meth)acrylatcopolymer-Schicht begegnet werden.

Mischungen zur Herstellung von Tabletten aus überzogenen Partikeln werden durch Vermischen der Pellets mit geeigneten Bindemitteln für die Tablettierung, nötigenfalls der Zugabe von zerfallsfördernden Substanzen und nötigenfalls der-Zugabe von Schmiermitteln zubereitet. Das Mischen kann in geeigneten Maschinen stattfinden. Ungeeignet sind Mischer, die zu Schäden an den überzogenen Partikeln führen, z. B. Pflugscharmischer. Zur Erzielung geeigneter kurzer ZerFallszeiten kann eine spezielle Reihenfolge bei der Zugabe der Hilfsstoffe zu den überzogenen Partikel erforderlich sein. Durch Vormischung mit der überzogenen Partikel mit dem Schmier oder Formentrennmittel Magnesiumstearat kann dessen Oberfläche hydrophobisiert und somit Verkleben vermieden werden.

Zum Tablettieren geeignete Mischungen enthalten üblicherweise 3 bis 15-Gew.-% eines Zerfallshilfsmittele, z. B. Kollidon CL und z.B. 0,1 bis 1 Gew.-% eines Schmier- und Formentrennmittels wie Magnesiumstearat. Der Bindemittelanteil bestimmt sich nach dem geforderten Anteil an überzogenen Partikeln.

Typische Bindemittel sind z. B. Cellactose^{®}, mikrokristalline Cellulose, Calciumphosphate, Ludipress^{®}, Lactose oder andere geeignete Zucker, Calciumsulfate oder Stärkederivate. Bevorzugt werden Substanzen mit geringer Schüttdichte.

Typische Zerfallshilfsmittel (Sprengmittel) sind quervernetzte Stärke- oder Cellulosederivate, sowie quervernetztes Polyvinylpyrrolidon. Ebenso sind Cellulosederivate geeignet. Durch Auswahl eines geeigneten Bindemittels kann die Verwendung von Zerfallshilfsmittel entfallen.

Typische Schmier und Formentrennmittel sind Magnesiumstearate oder andere geeignete Salze von Fettsäuren oder in der Literatur zu diesem Zweck aufgeführte Substanzen (z.B. Laurinsäure, Calciumstearat, Talkum usw.). Bei Verwendung geeigneter Maschinen (z.B. Tablettenpresse mit externer Schmierung) oder geeigneter Formulierungen kann die Verwendung eines Schmier- und Formentrennmittels in der Mischung entfallen.

Der Mischung kann gegebenenfalls ein Hilfsmittel zurfließverbesserung beigefügt sein (z. B. hochdisperse Kieselsäurederivate, Talkum usw.).

Das Tablettieren kann auf üblichen Tablettenpressen, Exzenter- oder Rundlauftablettenpressen erfolgen, bei Preßkräften im Bereich von 5 bis 40 kN, bevorzugt 10-20 kN. Die Tablettenpressen können mit Systemen zur externen Schmierung ausgestattet sein. Gegebenenfalls kommen spezielle Systeme zur Matrizenbefüllung zum Einsatz, die die Matrizenbefüllung mittels Rührflügeln vermeiden.

### Weitere multipartuläre Arzneiformen

Alternativ zu verpressten Tabletten bzw. Minitabletten, können die wirkstoffhaltigen, überzogenenen Pellets auch zu beliebige anderen, oral verabreichbareb multipartikuläre Arzneiformen verarbeitet werden. Die überzogenen Pellets können z. B. in Kapseln, z. B. Gelatinekapsel, verfüllt werden oder zu Sachets oder Trockensäfte formuliert werden.

### Verfahren zur Herstellung einer multipartikulären Arzneiform

Die Erfindung betrifft auch ein Verfahren zur Herstellung einer multipartikulären Arzneiform mit den Schritten
a) Erzeugen der wirkstoffhaltigen lipophilen Matrix durch Suspendieren und/oder Lösen des Wirkstoffs mit der oder den Substanzen, die die lipohile Matrix bilden und gegebenenfalls weiteren pharmazeutisch üblichen Hilfsstoffen durch intensives Mischen oder Schmelzen der Inhaltsstoffe,
b) Erzeugen von Präpellets (Pelletkerne) mittels Sprühauftrag des mucoadhaesiven Polymers in Mischung mit der wirkstoffhaltigen lipophilen Matrix auf einen Kern oder durch Rotagglomeration, Ausfällen oder Sprühverfahren ohne einen Kern, wobei z. B. das mucoadhaesive Polymer als Pulver vorlegt werden kann,
c) Erzeugen von Pellets durch Sprühauftrag eines Überzuges des anionischen Polymers oder Copolymers, das optional Beimengungen pharmazeutisch üblicher Hilfsstoffe, insbesondere Weichmachern und Trennmittel, enthalten kann, aus einer Dispersion oder organischer Lösung auf die Präpellets aus Schritt b),
d) Herstellung einer multipartikulären Arzneiform, durch Verfüllen oder Einarbeiten der Pellets aus Schritt c) in an sich bekannter Weise, gegebenenfalls unter Verwendung pharmazeutisch üblicher Hilfsstoffe, insbesondere durch Verarbeitung zu pellethaltigen Tabletten, Minitabletten, Kapseln, Sachets oder Trockensäften.

### Bevorzugtes Verfahren

Bevorzugt werden die Verfahrensschritte a) und b) wie folgt ausgeführt:
a) Erzeugen der inneren Matrix-Schicht durch Herstellen einer Emulsion, Dispersion oder Lösung des Wirkstoffs mit der oder den Substanzen für die lipohilen Matrix, und gegebenenfalls weiteren pharmazeutisch üblichen Hilfsstoffen durch intensives Mischen der Inhaltsstoffe in Wasser und Erzeugung einer Öl in Wasser-Präparation mit einer mittleren Teilchengröße von nicht mehr als 60, bevorzugt nicht mehr als 20 µm,
b) Erzeugen von Präpellets mittels Sprühauftrag der Öl in Wasser-Präparation aus Schritt a) auf das mucoadhaesive Polymer, das gegebenenfalls Beimengungen weiterer pharmazeutisch übliche Hilfsstoffe enthalten kann, und wobei die Inhaltstoffe in Form eines mikronisierten Pulvers, z. B. mit einer mittleren Korngröße von 10 bis 100 µm, vorliegen, durch Rotagglomeration, Extrusion oder Granulation.

### Vorteilhafte Wirkungen der Erfindung

Die erfindungsgemäße Arzneiform eignet sich zur gezielten und effektiven Freisetzung von wenig löslichen Wirkstoffen, wobei Peptide und Proteine einschließlich deren Derivate oder Konjugate ausgenommen sind. Die Arzneiform weist eine hohe Dosiersicherheit und verteilt sich gut im Darmlumen. Der enthaltene Wirkstoff wird dabei weitgehend gegenüber physikalischer, chemischer oder enzymatischer Inaktivierung geschützt und kann am definierten Wirkort so freigesetzt werden, daß ein hoher Anteil des Wirkstoffs vom Körper aufgenommen werden kann. Die Arzneiform kommt daher mit weniger Wirkstoff aus, da nur wenig Wirkstoff verloren geht. Die Gefahr von Nebenwirkungen wird durch die gezielte Abgabe insgesamt - verringert. Der Wirkort kann je nach therapeutischem Ziel variabel eingestellt werden. Der Zeitpunkt der Wirkstoffaufnahme kann somit besser gesteuert werden. Da es sich um eine orale Arzneiform handelt hat diese eine insgesamt bessere Akzeptanz der Patienten ("patient compliance") im Vergleich zu anderen Applikationsformen. Eine Vielzahl von wenig löslichen Wirkstoffen wird dadurch erstmals der peroralen Anwendung zugänglich und haben somit weniger Applikationsrisiken wie insbesondere bei parenteralen Applikationen. Auch die Kosten der Applikation können gering gehalten werden, da kein Fachpersonal für die Applikation notwendig ist.

Eine beschleunigte Freigabe bei gleichzeitiger Steigerung der Bioverfügbarkeit kann erreicht werden aus Matrixsystemen, in denen der Anteil des Polymeren mit mucoadhaesiver Wirkung in Gew.-% bevorzugt 2-fach, besonders bevorzugt 10 bis 200-fach höher ist als der Wirkstoffanteil.

### BEISPIELE

### Beispiele für Ausführungsformen mit wirkstoffhaltiger lipophiler Matrix

### 1. Beispiel:

### (Zidovudin; Löslichkeit in Wasser nach DAB 10 mindestens 50 Teile Wasser für 1 Teil Wirkstoff; entspr. 20 g / I).

### a) Vorbereitung der lipophilen Phase.

150 g Inwitor 312 (Schmelztemperatur 55-60 °C) werden in einem Wasserbad bei 65°C geschmolzen und 75 g Poloxamer 407 (Lutrol F127, Schmelztemperatur 50-55 °C) werden langsam in die Schmelze eingerührt. Das Wasserbad wird bis auf 52 °C heruntergekühlt und 12,5 g Tocopherol-acetat, und 5 g Na-Glycocholat unter Rühren zugegeben. Die Temperatur des Bades kann somit weitere 5°C gesenkt werden ohne dass das Fett wieder erstarrt. Die resultierende lipophile Matrix hat somit eine Schmelztemperatur von 38-41 °C und eine aus den einzelnen Komponenten errechnete Löslichkeit in Wasser nach DAB 10 von mindestens 40 Teilen Wasser für 1 Teil lipophile Matrix; entspr. 25 g/l). Zu dieser Lösung werden 500 g Zidovudin unter Rühren zugegeben.

### b) Herstellung einer Dispersion

1500 ml destilliertes Wasser werden zunächst bei 45 °C erhitzt und 30 g Na-Caprinat als Emulgator (2 %) zugegeben. Diese Lösung wird dann mit Zugabe von Zitronensäure auf einem pH-Werte von ca. 7 justiert. Zu dieser Lösung wird dann unter energischem Rühren die lipophile Phase dispergiert. Der Dispersions-Prozess kann beendet werden, wenn keine lipophile Teilchen nach mikroskopischer Untersuchung größer als 50-60 µm zu erkennen sind.

### c) Herstellung von muco-adhesiven Kernen

In einem GPCG1 mit Rotor Einsatz werden 700 g Na-Alginat Pulver, 285 g mikrokristalline Cellulose und 15 g Zitronensäure dazu gemischt. Die Dispersion beschrieben in b) wird als Bindemittel im Rotoagglomerationsprozess mit einer Sprührate von ca. 90 g/min gesprüht.
Der Rotor wird bei 1700-1800 UPM, die Zuluft auf 42 m³ / Stunde und die Temperatur der Luft auf 30 °C eingestellt.
Unter diesen Bedingungen können mucoadhaesive Kerne zwischen 250 und 600 µm mit einer Ausbeute bis zu 80 % produziert werden.
Eine therapeutische Dosierung von 250 mg ist in 886 mg Pelletkemen enthalten.

### d) Herstellung von überzogenen Pellets

Die Pelletkerne aus c) werden mittels gängiger Wirbelschichtverfahren mit EUDRAGIT® FS 30D* überzogen. Der Polymerauftrag beträgt 40 Gew.-% bezogen auf das Kemgewicht. Die Dispersion/Suspension zum Überziehen besteht aus:

| | |
|---|---|
| EUDRAGIT® FS 30 D | 44,65 % |
| Triethylcitrat | 0,67 % |
| Polysorbat 80 | 0,26 % |
| Glycerinmonostearat | 0,67 % |
| Wasser | 53,75 % |

| | |
|---|---|
| *= EUDRAGIT^{®} FS 30 D ist eine 30 %-ige wässrige Dispersion von EUDRAGIT^{®} FS: Copolymer aus 65 Gew.-% Methylacrylat, 25 Gew.% Methylmethacrylat und 10 Gew.-% Methacrylsäure. | |

Die somit erhaltenen Pellets können in einer Tablette mit üblichen pharmazeutischen Verfahren und Hilfsstoffen verpresst oder in Kapseln gefüllt werden.

### 2. Beispiel:

(Rosiglitazon; Löslichkeit in Wasser nach DAB 10 mindestens 1000 Teile Wasser für 1 Teil Wirkstoff; entspr. 1 g/l).

### a) Vorbereitung der lipophilen Phase

13 g Imwitor 312 (Schmelztemperatur 55 - 60°C) werden mit 4 g Poloxamer 407 (Lutrol F127, Schmelztemperatur 50-55 °C) bei 65°C im Wasserbad geschmolzen. Anschließend werden 1 g Caprylsäure, 1 g Na-Caprylat und 1 g Tocopherolacetat unter Rühren zugesetzt. Die resultierende lipophile Matrix hat somit eine Schmelztemperatur von 40-48 °C und eine aus den einzelnen Komponenten errechnete Löslichkeit in Wasser nach DAB 10 mindestens 700 Teile Wasser für 1 Teil lipophile Matrix (entspr. 1,5 g/l). Nach Abkühlen der Lösung auf 45°C werden 2,9 g Rosiglitazon unter schnellem Rühren in die lipophile Phase eingerührt und abgekühlt.

### b) Herstellung einer mucoadhaesiven Dispersion

20 g Chitosan werden in 1000 g Wasser dispergiert und anschließend unter sehr schnellem Rühren 20 g Citronensäure. In die erhaltene klare gelbliche viskose Lösung werden unter schnellem Rühren 2 g Na-Dodecanat zugesetzt und 1 h weitergerührt.

### c) Herstellung einer Sprühsuspension

Die erhaltene Dispersion aus a) wird mit dem Ultraturrax (20000 U/min) mit der Chitosan-Citrat Dispersion aus b) unter weiterer Abkühlung mit dem Eisbad bis auf 10°C mind. 10 min dispergiert. Der Dispergier-Prozess kann beendet werden, wenn keine lipophile Teilchen nach mikroskopische Untersuchung größer als 50-60 µm zu erkennen sind.

### c) Herstellung von mucoadhaesiven Kernen

Die Suspension aus c) wird mit dem GPCG1 (Glatt) mit einer Sprührate von 10 -12 g/min/kg auf 250 g Neutral-Pellets 400 - 600 µm bei einer Zulufttemperatur von 30°C und aufgesprüht. Die Zuluft wird hierbei auf 45 - 50m³/h eingestellt. Die Ausbeute liegt hierbei bei 90%. Eine therapeutische Dosierung von 8 mg ist in 179 mg Pelletkernen enthalten.

### d) Herstellung von überzogenen Pellets

Die so erhaltenen Pellets werden mittels gängiger Wirbelschichtverfahren mit EUDRAGIT® L12.5 überzogen. Der Polymerauftrag beträgt 40 Gew.% bezogen auf das Kemgewicht. Die Suspension zum Überziehen besteht aus:

| | |
|---|---|
| EUDRAGIT® L12.5 | 53,3% |
| Triethylcitrat | 1,33% |
| Isopropanol | 38,3% |
| Talkum | 2,0% |
| Wasser | 5,0% |

Die somit erhaltenen Pellets können in einer Tablette mit üblichen pharmazeutischen Verfahren und Hilfsstoffen verpresst oder in Kapseln gefüllt werden.

### 3. Beispiel:

(Oxcarbazepin; Löslichkeit in Wasser nach DAB 10 mindestens 10000 Teile Wasser für 1 Teil Wirkstoff; entspr. 0,1 g/l)

### a) Vorbereitung der lipophilen Phase

200 g Imwitor 312 (Schmelztemperatur 55 - 60°C) und 400 g Dynasan 114 (Schmelztemperatur 55 - 58°C) werden mit 30 g Tocopherolacetat bei 65°C geschmolzen und in einen Granulator (Bohle) gegeben. Dazu werden 20 g Na-Caprylat unter Rühren zugegeben. Die Mischung wird auf 45 °C abgekühlt und darin 940 g Oxcarbazepin gelöst. Die resultierende lipophile Matrix hat somit eine Schmelztemperatur von 39-46 °C und eine aus den einzelnen Komponenten errechnete Löslichkeit in Wasser nach DAB 10 mindestens 7000 Teilen Wasser für 1 Teil lipophile Matrix. Die lipophile Matrix wird unter Kühlung auf eine Teilchengröße unter 50µm gemahlen.

### b) Herstellung einer Pufferlösung

1 g Na-Citrat und 1 g Citronensäure werden in 500 g Wasser gelöst. Unter schnellem Rühren werden 0,5 g Na-Cholat zugegeben.

### c) Granulation

Die gemahlene wirkstoffhaltige lipophile Matrix aus a) wird in einem Granulator mit 1500 g Blanose 7LF gemischt. Anschließend wird mit der wäßrigen Pufferlösung aus b) granuliert, so dass 0,2 bis 0,5 mm große Partikel entstehen, die auf einem Spheronizer ausgerundet werden. Die erhaltenen feuchten Kerne werden bei 30 bis 25 °C in einem Wirbelschichttrockner milde getrocknet. Eine therapeutische Dosierung von 300 mg ist in 837 mg Pelletkernen enthalten.

### d) Herstellung von überzogenen Pellets

Die so hergestellten Kerne aus c) werden mittels gängiger Wirbelschichtverfahren mit EUDRAGIT® FS 30D überzogen. Der Polymerauftrag beträgt 40 Gew.-% bezogen auf das Kemgewicht. Die Dispersion/Suspension zum Überziehen besteht aus:

| | |
|---|---|
| EUDRAGIT® FS 30 D | 44,65 % |
| Triethylcitrat | 0,67 % |
| Polysorbat 80 | 0,26 % |
| Glycerinmonostearat | 0,67 % |
| Wasser | 53,75 % |

Die somit erhaltenen Pellets können zu einer Tablette mit üblichen pharmazeutischen Verfahren und Hilfsstoffen verpresst oder in Kapseln gefüllt werden.

## Patentansprüche

1. Orale multipartikuläre Arzneiform, in Form eines Behältnisses, das im pH-Bereich des Magens zerfällt, enthaltend eine Vielzahl von Pellets, Partikeln, Granulaten oder Agglomeraten mit einem mittleren Durchmesser im Bereich von 50 bis 2500 µm, die im wesentlichen aufgebaut sind aus
a) einer inneren Matrix-Schicht, enthaltend einen Wirkstoff, der kein Peptid oder ein Protein einschließlich deren Derivate oder Konjugate ist, eine lipophile Matrix, die einen Schmelzpunkt oberhalb von 37 °C aufweist, und ein Polymer mit mucoadhaesiver Wirkung,
b) einem äußeren verfilmten Überzug, bestehend im wesentlichen aus einem anionischen Polymeren oder Copolymeren, das optional mit pharmazeutisch üblichen Hilfsstoffen formuliert sein kann,
**dadurch gekennzeichnet, dass** der Wirkstoff eine Löslichkeit in Wasser nach DAB 10 von mindestens 30 Volumenteilen Wasser für einen Gewichtsteil Wirkstoff aufweist und in die lipophile Matrix eingebettet ist und die wirkstoffhaltige lipophile Matrix in eine Matrix aus dem Polymeren mit mucoadhaesiver Wirkung eingebettet ist, die ein Chitosan enthält und mittels einer Säure oder einem Puffersystem auf pH 5,0 bis 5,5 eingestellt wird und mit einem äußeren verfilmten Überzug kombiniert wird, der sich im Bereich von pH 6,0 bis 8,0 aufzulösen beginnt.

2. Arzneiform nach Anspruch 1, **dadurch gekennzeichnet, daß** sich der Wirkstoff und die Substanz oder die Substanzen, die die lipophile Matrix bilden, sich in ihrer Löslichkeit in Wasser nach DAB 10 und nicht mehr als +/- 50 % unterscheiden und/oder in ihrem Verteilungskoeffizienten nach Anhang V zu RL 67/548/EWG, A.8, nach nicht mehr als +/- 60 % unterscheiden und/oder in ihrem HLB-Wert gemessen nach Marszall nicht mehr als +/- 80 % unterscheiden.

3. Arzneiform nach Anspruch 1 oder 2, **dadurch gekennzeichnet, daß** die Substanz oder die Substanzen, die die lipophile Matrix bilden, und das Polymer mit mucoadhaesiver Wirkung entweder die gleiche ionische Eigenschaft aufweisen oder dass im Falle entgegengesetzter ionischer Eigenschaften, das Polymer mit mucoadhaesiver Wirkung zu mindestens 50 % in neutralisierter Form vorliegt.

4. Arzneiform nach einem oder mehreren der Ansprüche 1 bis 3 , **dadurch gekennzeichnet, daß** die lipophile Matrix zu 80 bis 100 Gew.-% aus einer Substanz mit einem HLB-Wert von 0 bis 15 oder einer Mischung von Substanzen mit einem gemittelten HLB-Wert von 0 bis 15 besteht und 0 bis 20 Gew.-% an pharmazeutisch üblichen Hitfsstoffen, insbesondere Stabilisatoren, Verdickungsmittel oder Adsorbentien, enthalten kann.

5. Arzneiform nach einem oder mehreren der Ansprüche 1 bis 4, **dadurch gekennzeichnet, daß** die Substanz oder die Substanzen, die die lipophile Matrix bilden, zu der Gruppe der Öle, Fette. Mono-, Di- oder Triglyceride, Fettsäuren, Fettalkohole, insbesondere C₅- bis C₂₀-Fettsäure und/oder einen C₆- bis C₂₀-Alkohol einschließlich deren Salze, Ether-, Ester oder Amid-Derivaten, Phospholipide, Lecithine, Emulgatoren, Lipoide, lipidlösliches Vitamine oder Tenside gehören.

6. Arzneiform nach einem oder mehreren der Ansprüche 1 bis 5, **dadurch gekennzeichnet, daß** die lipophile Matrix eine der folgenden Lipidpräparationen enthält: (Imwitor 308) Glycerylmonocaprylate mit einem Monoesteranteil >80 %, (Imwitor 312) Glycerylmonolaurate mit enem Monoesteranteil > 90 %, (Imwitor 491) Glycerolmonosterate (C₁₈ + C₁₈) mit einem Monoesteranteil >90 %, (Imwitor 900 P) Glycerolmonosterat mit einem Monoesteranteil von 40 - 55% und einem C₁₈-Gehalt von 40 - 60%, (Imwitor 900 K) Glycerolmonosterat, mit einem Monoesteranteil von 40 - 55 % und einem C₁₈-Gehalt von 60 -80 %, (Imwitor 742) Mittelkettige C₈ und C₁₀ Glyceride mit einem Monoesteranteil von 45 - 55 %, (Imwitor 928) Partielle Glyceride gesättigter pflanzlicher C₁₀-C₁₈Fettsäuren mit einem Hauptanteil an C₁₂, und mit einem Monoesteranteil von 34-36%, C₈ and C₁₀-Glyceride, NaCaprylat oder NaCapriat.

7. Arzneiform nach einem oder mehreren der Ansprüche 1 bis 6 , **dadurch gekennzeichnet, daß** der Wirkstoff zu mindestens 10 % in der lipophilen Matrix löslich ist.

8. Arzneiform nach einem oder mehreren der Ansprüche 1 bis 7, **dadurch gekennzeichnet, daß** der Gehalt der virkstoffhaltigen lipophilen Matrix an der inneren Matrix-Schicht a) 5 bis 60 Gew.-%. beträgt.

9. Arzneiform nach einem oder mehreren der Ansprüche 1 bis 8, **dadurch gekennzeichnet, daß** das Behältnisses, das im pH-Bereich des Magens zerfällt, eine Kapsel, eine Tablette, eine Trockensaftformulierung oder ein Sachet ist.

10. Arzneiform nach einem oder mehreren der Ansprüche 1 bis 9, **dadurch gekennzeichnet, daß** der äußere Überzug durch die Wahl des anionischen Polymeren oder Copolymeren bzw. seiner Formulierung mit Hilfsstoffen und seiner Schichtdicke so eingestellt ist, daß sich dieser in pH-Bereichen von 4,0 bis 8,0 im Darm Innerhalb 10 bis 60 min auflöst, so daß die wirkstoffhaltige lipophile Matrix, die in der eingebettet mucoadhaesive Matrix-Schicht frei wird, an die Darmmucosa binden und dort den Wirkstoff freisetzen kann, wobei das Polymere mit mucoadhaesiver Wirkung so gewählt ist, daß es in einem Bereich +/- 0,5 pH-Einheiten bezogen auf den pH-Wert, bei dem sich der äußere überzug aufzulösen beginnt, eine mucoadhaesive Wirkung von ηb**=** 150 bis 1000 mpa-s und eine Wasseraufnahme von 10 bis 750 % in 15 min aufweist und der Wirkstoffanteil der lipophilen Matrix maximal 90 Gew.-% beträgt,

11. Arzneiform nach einem oder mehreren der Ansprüche 1 bis 10, **dadurch gekennzeichnet, daß** der äußere verfilmte Überzug Celluloseglycolat (Duodcell®), Celluloseacetatphtalat (CAP, Cellulosi acetas, PhEur, Celluloseacetate-phtalate, NF, Aquateric®), Celluloseacetatsuccinat (CAS), Celluloseacetattrimeliat (CAT). Hydroxypropylmethylcellulosephtalat (HPMCP, HP50. HP55), Hydroxypropylmethylcelluloseacetatsuccinat (HPMCAS -LF, -MF, -HF), Polyvinylacetatphtalat (PVAP, Sureteric®), Vinylacetat-Vinylpyrolidon-Copolymer (PVAc, Kollidon® VA64), Vinylacetat:Crotonsäure-Copolymer 9:1 (VAC:CRA, Kollicoat® VAC) und/oder Sheillack ist.

12. Arzneiform nach einem oder mehreren der Ansprüche 1 bis 10, **dadurch gekennzeichnet, daß** der äußere verfilmte Überzug aus einem (Meth)acrylat-Copolymeren mit einem Gehalt an Monomeren mit anionischen Gruppen von 5 bis 60 Gew.% besteht.

13. Arzneiform nach einem oder mehreren der Ansprüche 1 bis 12, **dadurch gekennzeichnet, daß** die Schichtdicke des äußeren Überzuges im Bereich von 20 bis 200 µm liegt.

14. Arzneiform nach einem oder mehreren der Ansprüche 1 bis 13, **dadurch gekennzeichnet, daß** die innere Schicht a) einen Efflux-Pumpen-Inhibitor und/oder einen Penetrationsförderer enthält.

15. Arzneiform nach einem oder mehreren der Ansprüche 1 bis 14, **dadurch gekennzeichnet, daß** zwischen der inneren Schicht a) und der äußeren vefilmten Überzugsschicht b) eine Trennschicht aufgebracht ist.

16. Arzneiform nach einem oder mehreren der Ansprüche 1 bis 15, **dadurch gekennzeichnet, dass** der in der lipophilen Matrix fomulierte Wirkstoff aus der Gruppe der BCS-Klassen II und IV (Bio-Pharmaceutical-Classification-System nach Prof. Amidon) und/oder aus der Gruppe der Antiandrogene, Antidepressiva, Antidiabetika, Antirheumatika, Glucocorticoide, Cytostatika, Migränemittel, Neuroleptika, Antibiotika, Östrogene, Vitamine, Psychopharmaca, ACE-Hemmer, β-Blocker, Ca-Kanalblocker, Diuretika, Herzglykoside, Antiepileptika, Diuretika/Antiglaukom, Urikostatika, H₂-Rezeptorenblocker und Virostatika ausgewählt ist

17. Arzneiform nach einem oder mehreren der Ansprüche 1 bis 16, **dadurch gekennzeichnet, dass** der in der lipophilen Matrix fomulierte Wirkstoff enthaltene Wirkstoff Bicalutamid, Anastrozol, Glimiprid, Nilutamid, Bromocriptin, Ketotifen, Letrozol, Naratriptan, Gancidovir, Orlistat, Mesoprosztol, Granistron, Pioglitazon, Lamivudin. Rosiglitazon, Zidovudin, Enalapril, Atenolol, Nadolol, Felodipin, Bepridil, Furosemid, Digoxin, Digitoxin, Carbamazepin, Acetazoiamid, Allopurinol, Cimetidin, Ranitidin oder Oxcarbazepin ist.

18. Verfahren zur Herstellung einer multipartikulären Arzneiform nach einem oder mehreren der Ansprüche 1 bis 17, mit den Schritten
a) Erzeugen der wirkstoffhaltigen lipophilen Matrix durch Suspendieren und/oder Lösen des Wirkstoffs mit der oder den Substanzen, die die lipophile Matrix bilden und gegebenenfalls weiteren pharmazeutisch üblichen Hilfsstoffen durch intensives Mischen oder Schmelzen der inhaltsstoffe,
b) Erzeugen von Präpellets (Pelletkerne) mittels Sprühauftrag des mucoadhaesiven Polymers in Mischung mit der wirkstoffhaltigen lipophilen Matrix auf einen Kern oder durch Rotagglomeration, Ausfällen oder Sprühverfahren ohne einen Kern,
c) Erzeugen von Pellets durch Sprühauftrag eines Überzuges des anionischen Polymers oder Copolymers, das optional Beimengungen pharmazeutisch üblicher Hilfsstoffe, insbesondere Weichmachern und Trennmittel; enthalten kann, aus einer Dispersion oder organischer Lösung auf die Präpellets aus Schritt b).
d) Herstellung einer multipartikulären Arzneiform, durch Verfüllen oder Einarbeiten der Pellets aus Schritt c) in an sich bekannter Weise, gegebenenfalls unter Verwendung pharmazeutisch üblicher Hilfsstoffe, insbesondere durch Verarbeitung zu pellethaltigen Tabletten, Minitabletten, Kapseln, Sachets oder Trockensäften.

19. Verfahren zur Herstellung einer multipartikulären Arzneiform nach Anspruch 18, **dadurch gekennzeichnet, daß** die Schritte a) und b) wie folgt ausgeführt werden
a) Erzeugen der inneren Matrix-Schicht durch Herstellen Emulsion, Dispersion oder Lösung des Wirkstoffs mit der oder den Substanzen für die lipophilen Matrix, und gegebenenfalls weiteren pharmazeutisch üblichen Hilfsstoffen durch intensives Mischen der Inhaltsstoffe in Wasser und Erzeugung einer Öl in WasserPräparation mit einer mittleren Teilchengröße von nicht mehr als 60 µm,
b) Erzeugen von Präpellets mittels Sprühauftrag der Öl in WasserPräparation aus Schritt a) auf das mucoadhaesive Polymer, das gegebenenfalls Beimengungen weiterer pharmazeutisch üblicher Hitfsstoffe enthalten kann, und wobei die Inhaltsstoffe in Form eines mikronisierten Pulvers vorliegen, durch Rotoagglomeration, Extrusion oder Granulation.

## Claims

1. Oral multiparticle pharmaceutical form in the form of a casing that disintegrates in the gastric pH range and contains numerous pellets, particles, granules or agglomerates with a mean diameter of 50-2500 µm, which are essentially composed of:
a)an inner matrix layer comprising an active substance other than a peptide or a protein and their derivatives or conjugates; a lipophilic matrix with a melting point of over 37°C; and a mucoadhesive polymer,
b) a film-applied outer coating, essentially consisting of an anionic polymer or copolymer, which can be optionally formulated with pharmaceutically customary excipients,
**characterized in that**
the active substance has a water-solubility as defined in the German Pharmacopeia DAB 10 of at least 30 parts by volume of water for 1 part by weight of the active substance and is embedded in the lipophilic matrix, and the lipophilic matrix with its active substance is embedded in a matrix made from the mucoadhesive polymer, which matrix comprises a chitosan and is adjusted to pH 5.0-5.5 by means of an acid or a buffer system, and is combined with a film-applied outer coating which begins to dissolve in the range of pH 6.0 to 8.0.

2. Pharmaceutical form according to Claim 1, **characterized in that** the active substance and the substance or substances forming the lipophilic matrix do not differ from each other in their water-solubility as defined in the German Pharmacopeia DAB 10 by more than ± 50%, in their distribution coefficient defined in Annexe V to EU Directive No. 67/548/EEC Point A.8 by more than ± 60%, and/or in their HLB value as measured by Marszall's method by more than ± 80%.

3. Pharmaceutical form according to Claim 1 or 2, **characterized in that** the mucoadhesive polymer and the substance or substances forming the lipophilic matrix either have the same ionic character, or - if they have opposite ionic characters - the mucoadhesive polymer is present in a form neutralized to an extent of at least 50%.

4. Pharmaceutical form according to one or more of Claims 1-3, **characterized in that** 80-100 wt-% of the lipophilic matrix is formed by a substance with an HLB value of 0-15 or by a mixture of substances with a mean HLB value of 0-15, and can contain 0-20 wt-% of pharmaceutically customary excipients, especially stabilizers, thickeners or adsorbents.

5. Pharmaceutical form according to one or more of Claims 1-4, **characterized in that** the substance or substances forming the lipophilic matrix belong to the group of oils, fats, mono-, di- or triglycerides, fatty acids, fatty alcohols, especially C₅-C₂₀ fatty acid and/or a C₅-C₂₀ alcohol, including their salts, ether derivatives, ester derivatives or amide derivatives, phospholipids, lecithins, emulsifiers, lipoids, fat-soluble vitamins or surfactants.

6. Pharmaceutical form according to one or more of Claims 1-5, **characterized in that** the lipophilic matrix contains one of the following lipid preparations: Imwitor 308 (glyceryl monocaprylate containing over 80% of the monoester), Imwitor 312 (glyceryl monolaurate containing over 90% of the monoester), Imwitor 491 [glyceryl monostearate (C₁₆ + C₁₈) containing over 90% of the monoesters], Imwitor 900 P (glyceryl monostearate containing 40-55% of the monoester and 40-60% of C₁₈ compound), Imwitor 900 K (glyceryl monostearate containing 40-55% of the monoester and 60-80% of C₁₈ compound), Imwitor 742 (medium-chain C₈ and C₁₀ glycerides containing 45-55% of monoesters), Imwitor 928 (partial glycerides formed with saturated C₁₀-C₁₈ fatty acids of plant origin, where the main component is C₁₂ compound and the monoester content is 34-36%), C₈ and C₁₀ glycerides, sodium caprylate or sodium caprate.

7. Pharmaceutical form according to one or more of Claims 1-6, **characterized in that** the active substance is soluble in the lipophilic matrix to an extent of at least 10%.

8. Pharmaceutical form according to one or more of Claims 1-7, **characterized in that** the amount of the inner matrix layer a) in the lipophilic matrix that contains the active substance is 5-60 wt-%.

9. Pharmaceutical form according to one or more of Claims 1-8, **characterized in that** the casing that disintegrates in the gastric pH range is a capsule, a tablet, a reconstitutable powder formulation or a sachet.

10. Pharmaceutical form according to one or more of Claims 1-9, **characterized in that** the properties of the outer coating are adjusted by the appropriate choice of the anionic polymer or copolymer and formulation or of its excipients with and of its layer thickness, in such a way that it - the outer coating - dissolves in the gut at a pH of 4.0-8.0 within 10-60 minutes, so that the lipophilic matrix embedded - with its active substance - in the mucoadhesive matrix layer is exposed, can bind to the intestinal mucosa and can release the active substance there; the mucoadhesive polymer is so chosen that, when the pH is within ± 0.5 pH units from the pH value at which the outer coating begins to dissolve, it possesses a mucoadhesive action n_{b} of 150-1000 mPa.sec and a water absorption of 10-750% in 15 minutes, and the active substance constitutes at most 90 wt-% of the lipophilic matrix.

11. Pharmaceutical form according to one or more of Claims 1-10, **characterized in that** the film-applied outer coating is cellulose glycolate (Duodcell®), cellulose acetate phthalate (CAP, *"Cellulosi acetas"* as per European Pharmacopeia, cellulose acetate phthalate as per NF, or Aquateric®), cellulose acetate succinate (CAS), cellulose acetate trimellitate (CAT), hydroxy propyl methyl cellulose phthalate (HPMCP, HP50, HP55), hydroxy propyl methyl cellulose acetate succinate (HPMCAS-LF, -MF -HF), polyvinyl acetate phthalate (PVAP or Sureteric^{®}), vinyl acetate-vinylpyrrolidone copolymer (PVAc or Kollidon^{®} VA64), a 9:1 vinyl acetate:crotonic acid copolymer (VAC:CRA or Kollicoat^{®} VAC) and/or shellac.

12. Pharmaceutical form according to one or more of Claims 1-10, **characterized in that** the film-applied outer coating consists of a (meth)acrylate copolymer containing 5-60 wt-% of monomers with anionic groups,

13. Pharmaceutical form according to one or more of Claims 1-12, **characterized in that** the layer thickness of the outer coating is from 20 to 200 µm.

14. Pharmaceutical form according to one or more of Claims 1-13, **characterized in that** the inner layer a) contains an efflux pump inhibitor and/or a penetration promoting agent.

15. Pharmaceutical form according to one or more of Claims 1-14, **characterized in that** there is a separating layer applied between the inner layer a) and the film-applied outer coating layer b).

16. Pharmaceutical form according to one or more of Claims 1-15, **characterized in that** the active substance formulated in the lipophilic matrix is chosen from the group of Classes II and IV of the Bio-Pharmaceutical Classification System (BCS) of Prof. Amidon and/or from the group of antiandrogens, antidepressants, antidiabetics, antirheumatics, glucocorticoids, cytostatics, antimigraine drugs, neuroleptics, antibiotics, estrogens, vitamins, pyschopharmaceuticals, ACE inhibitors, beta-blockers, calcium channel blockers, diuretics, cardiac glycosides, antiepileptics, diuretics/antiglaucoma agents, uricostatics, H₂ receptor blockers and virostatics.

17. Pharmaceutical form according to one or more of Claims 1-16, **characterized in that** the active substance formulated in the lipophilic matrix is bicalutamide, anastrozole, glimepiride, nilutamide, bromocriptine, ketotifen, letrozole, naratriptan, ganciclovir, orlistat, mesoprostol, granisetron, pioglitazone, lamivudine, rosiglitazone, zidovudine, enalapril, atenolol, nadolol, felodipine, bepridil, furosemide, digoxin, digitoxin, carbamazepine, acetazolamide, allopurinol, cimetidine, ranitidine or oxcarbazepine.

18. Process for the preparation of a multiparticle pharmaceutical form according to one or more of Claims 1-17, comprising the following steps:
a) the lipophilic matrix containing the active substance is prepared by suspending and/or dissolving the active substance with the substance or substances forming the lipophilic matrix, possibly together with other pharmaceutically customary excipients, by intense mixing or melting of the constituents,
b) prepellets (pellet cores) are prepared by spraying the mucoadhesive polymer, mixed with the lipophilic matrix that contains the active substance, onto a core, or by rotary agglomeration, precipitation or spraying without any core,
c) pellets are produced by spraying the prepellets obtained in step b), from a dispersion or organic solution, with a coating of the anionic polymer or copolymer, optionally containing admixtures of pharmaceutically customary excipients, especially plasticizers and separating agents,
d) a multiparticle pharmaceutical form is prepared by introducing or incorporating the pellets obtained in step c) in a conventional way, possibly using pharmaceutically customary excipients, especially by processing the composition into pellet-containing tablets, minitablets, capsules, sachets or reconstitutable powders.

19. Process for the preparation of a multiparticle pharmaceutical form according to Claim 18, **characterized in that** steps a) and b) are carried out as follows
a) the inner matrix layer is made by preparing an emulsion, dispersion or solution of the active substance with the one or more substances for the lipophilic matrix and possibly with other pharmaceutically customary excipients, by intensely mixing the constituents in water and preparing an oil-in-water system with an average particle size of at most 60 µm,
b) prepellets are prepared by spraying the oil-in-water system obtained in step a) onto the mucoadhesive polymer, which may also contain admixtures of pharmaceutically customary excipients, where the ingredients are present in the form of a micronized powder, by rotary agglomeration, extrusion or granulation.

## Revendications

1. Forme galénique multiparticulaire orale, sous forme d'un récipient, qui se décompose dans le domaine de pH de l'estomac, contenant un certain nombre de pastilles, particules, granulés ou agglomérats avec un diamètre moyen situé dans la plage allant de 50 à 2500 *µ*m, qui sont élaborés essentiellement à partir de :
a) une couche de matrice interne, contenant un agent actif, qui n'est pas un peptide ou une protéine, y compris leurs dérivés ou conjugués, une matrice lipophile, qui présente un point de fusion supérieur à 37°C et un polymère avec action mucoadhésive,
b) un enduit pelliculé externe, consistant essentiellement en un polymère ou copolymère anionique, qui peut être formulé le cas échéant avec des auxiliaires pharmaceutiques usuels,
**caractérisée en ce que** l'agent actif présente une solubilité dans l'eau selon DAB 10 d'au moins 30 parties en volume d'eau pour une partie en poids d'agent actif et est incorporé dans la matrice lipophile et **en ce que** la matrice lipophile contenant l'agent actif est incorporée dans une matrice en le polymère avec action mucoadhésive, qui contient un chitosan et qui est ajustée à un pH de 5, 0 à 5,5 avec un acide ou un système tampon et est combinée à un enduit pelliculé externe, qui commence à se dissoudre dans la gamme de pH allant de 6,0 à 8,0.

2. Forme galénique selon la revendication 1, **caractérisée en ce que** l'agent actif et la ou les substances qui forment la matrice lipophile, ne se différencient pas de plus de ± 50 % au niveau de leur solubilité dans l'eau selon DAB 10 et/ou ne se différencient pas de plus de ± 60 % au niveau de leur coefficient de distribution selon l'annexe V de RL 67/548/EWG, A.B. et/ou ne se différencient pas de plus de ± 80 % au niveau de leur valeur EHL, mesurée selon Marszall.

3. Forme galénique selon la revendication 1 ou 2, **caractérisée en ce que** la ou les substances qui forment la matrice lipophile, et le polymère avec action mucoadhésive présentent la même propriété ionique ou **en ce que** dans le cas où les propriétés ioniques sont opposées, le polymère avec action mucoadhésive se présente pour au moins 50 %, sous forme neutralisée.

4. Forme galénique selon l'une ou plusieurs des revendications 1 à 3, **caractérisée en ce que** la matrice lipophile consiste pour 80 à 100 % en poids en une substance avec une valeur EHL allant de 0 à 15 ou un mélange de substances avec une valeur EHL moyenne allant de 0 à 15 et peut contenir 0 à 20 % en poids d'auxiliaires pharmaceutiques usuels, en particulier de stabilisants, agents épaississants ou adsorbants.

5. Forme galénique selon l'une ou plusieurs des revendications 1 à 4, **caractérisée en ce que** la ou les substances qui forment la matrice lipophile, appartiennent au groupe des huiles, graisses, monoglycérides, diglycérides ou triglycérides, acides gras, alcools gras, en particulier acides gras en C₅ à C₂₀ et/ou un alcool en C₅ à C₂₀, y compris leurs sels, dérivés éther, ester ou amide, phospholipides, lécithines, émulsionnants, lipoïdes, vitamines liposolubles ou tensioactifs.

6. Forme galénique selon l'une ou plusieurs des revendications 1 à 5, **caractérisée en ce que** la matrice lipophile contient une des préparations suivantes de lipides : (Imwitor 308) monocaprylates de glycéryle avec une proportion de monoester > 80 %, (Imwitor 312) monolaurates de glycéryle avec une proportion de monoester > 90 %, (Imwitor 491) monostéarates de glycérol (C₁₆ + C₁₈) avec une proportion de monoester > 90 %, (Imwitor 900P) monostéarate de glycérol avec une proportion de monoester de 40-55 % et une teneur en C₁₈ de 40-60 %, (Imwitor 900K) monostéarate de glycérol avec une proportion de monoester de 40-55 % et une teneur en C₁₈ de 60-80 %, (Imwitor 742) glycérides à chaîne moyenne C₈ et C₁₀ avec une proportion de monoester de 45-55 %, (Imwitor 928) glycérides partiels d'acides gras végétaux saturés en C₁₀-C₁₈ avec une proportion principale en C₁₂ et avec une proportion de monoester de 34-36 %, des glycérides en C₈ et C₁₀, le caprylate de Na ou le caprate de Na.

7. Forme galénique selon l'une ou plusieurs des revendications 1 à 6, **caractérisée en ce que** l'agent actif est soluble à au moins 10 % dans la matrice lipophile.

8. Forme galénique selon l'une ou plusieurs des revendications 1 à 7, **caractérisée en ce que** la teneur de la matrice lipophile contenant l'agent actif dans la couche de matrice interne a) se situe dans l'intervalle allant de 5 à 60 % en poids.

9. Forme galénique selon l'une ou plusieurs des revendications 1 à 8, **caractérisée en ce que** le récipient, qui se décompose dans le domaine de pH de l'estomac, est une capsule, un comprimé, une formulation de suc sec ou un sachet.

10. Forme galénique selon l'une ou plusieurs des revendications 1 à 9, **caractérisée en ce que** l'enduit externe est ajusté par le choix du polymère ou copolymère anionique et respectivement, de sa formulation avec des auxiliaires et son épaisseur de couche, de sorte que celui-ci se dissout dans la gamme de pH allant de 4,0 à 8,0 de l'intestin, en 10 à 60 minutes, de sorte que la matrice lipophile contenant l'agent actif, qui est libre dans la couche de matrice mucoadhésive incorporée, peut se lier à la muqueuse intestinale et y libérer l'agent actif, où le polymère avec action mucoadhésive est choisi de sorte qu'il présente, dans une gamme de pH de ± 0,5 unité sur base du pH auquel l'enduit externe commence à se dissoudre, une action mucoadhésive de η_{b} = 150 à 1000 mPa.s et une absorption d'eau de 10 à 750 % en 15 minutes, et la proportion d'agent actif de la matrice lipophile s'élève à maximum 90 % en poids.

11. Forme galénique selon l'une ou plusieurs des revendications 1 à 10, **caractérisée en ce que** l'enduit pelliculé externe est le glycolate de cellulose (Duodcell^{®}), l'acétophtalate de cellulose (CAP, Cellulosi acetas, PhEur, le phtalate d'acétate de cellulose, NF, Aquateric^{®}), l'acétosuccinate de cellulose (CAS), l'acétotrimellitate de cellulose (CAT), le phtalate d'hydroxypropylméthylcellulose (HPMCP, HP50, HP55), l'acétosuccinate d'hydroxypropylméthylcellulose (HPMCAS-LF, -MF, -HF), le poly(acétophtalate de vinyle) (PVAP, Sureteric^{®}), le copolymère acétate de vinyle-vinylpyrrolidone (PVAc, Kollidon^{®} VA64), le copolymère acétate de vinyle-acide crotonique 9:1 (VAC:CRA, Kollicoat^{®} VAC) et/ou une gomme-laque.

12. Forme galénique selon l'une ou plusieurs des revendications 1 à 10, **caractérisée en ce que** l'enduit pelliculé externe consiste en un copolymère de (méth)acrylate avec une teneur en monomères avec groupes anioniques allant de 5 à 60 % en poids.

13. Forme galénique selon l'une ou plusieurs des revendications 1 à 12, **caractérisée en ce que** l'épaisseur de couche de l'enduit externe se situe dans la plage allant de 20 à 200 µm.

14. Formé galénique selon l'une ou plusieurs des revendications 1 à 13, **caractérisée en ce que** la couche interne a) contient un inhibiteur des pompes d'efflux et/ou un agent accélérant la pénétration.

15. Forme galénique selon l'une ou plusieurs des revendications 1 à 14, **caractérisée en ce qu'**entre la couche interne a) et la couche d'enduit pelliculé externe b), une couche de séparation est appliquée.

16. Forme galénique selon l'une ou plusieurs des revendications 1 à 15, **caractérisée en ce que** l'agent actif formulé dans la matrice lipophile est choisi parmi le groupe des classes BCS II et IV (système de classification bio-pharmaceutique du professeur Amidon) et/ou parmi le groupe des antiandrogènes, antidépresseurs, antidiabétiques, antirhumatismaux, glucocorticoïdes, cytostatiques, antimigraineux, neuroleptiques, antibiotiques, oestrogènes, vitamines, psychopharmaceutiques, inhibiteurs d'ACE, β-bloquants, agents bloquant le canal Ca, diurétiques, glycosides cardiaques, antiépileptiques, diurétiques/antiglaucome, uricostatiques, agents bloquant le récepteur H₂ et virostatiques.

17. Forme galénique selon l'une ou plusieurs des revendications 1 à 16, **caractérisée en ce que** l'agent actif formulé dans la matrice lipophile est le bicalutamide, l'anastrozol, le glimipride, le nilutamide, la bromocriptine, le kétotiféne, le letrozol, le naratriptan, le ganciclovir, l'orlistat, le misoprostol, le granistrone, le pioglitazon, la lamivudine, le rosiglitazone, la zidovudine, l'énalapril, l'aténolol, le nadolol, la félodipine, le bépridil, le furosémide, la digoxine, la digitoxine, la carbamazépine, l'acétozolamide, l'allopurinol, la cimétidine, la ranitidine ou l'oxacarbazépine.

18. Procédé de préparation d'une forme galénique multiparticulaire selon l'une ou plusieurs des revendications 1 à 17, comportant les étapes de :
a) production de la matrice lipophile contenant l'agent actif par mise en suspension et/ou dissolution de l'agent actif avec la ou les substances, qui forment la matrice lipophile et le cas échéant, d'autres auxiliaires pharmaceutiques usuels, par mélange important ou fusion des constituants,
b) production de prépastilles (coeurs de pastille) par application par pulvérisation du polymère mucoadhésif en mélange avec la matrice lipophile contenant l'agent actif, sur un coeur ou par agglomération rotative, précipitation ou procédé de pulvérisation sans coeur,
c) production de pastilles par application par pulvérisation d'un enduit du polymère ou copolymère anionique, qui peut contenir des quantités facultatives d'auxiliaires pharmaceutiques usuels, en particulier des plastifiants et agents de séparation, à partir d'une dispersion ou solution organique sur les prépastilles de l'étape b),
d) préparation d'une forme galénique multiparticulaire par remplissage ou incorporation des pastilles de l'étape c), de manière connue en soi, le cas échéant en utilisant des auxiliaires pharmaceutiques usuels, en particulier par traitement en comprimés, minicomprimés, capsules, sachets ou sucs secs contenant des pastilles.

19. Procédé de préparation d'une forme galénique multiparticulaire selon la revendication 18, **caractérisé en ce que** les étapes a) et b) sont réalisées de la manière suivante:
a) production de la couche de matrice interne par préparation d'une émulsion, dispersion ou solution de l'agent actif avec la ou les substances pour la matrice lipophile et le cas échéant, d'autres auxiliaires pharmaceutiques usuels, par mélange important des constituants dans l'eau ou production d'une préparation huile dans eau, avec une taille moyenne des particules non supérieure à 60 µm,
b) production de prépastilles par application par pulvérisation de la préparation huile dans eau de l'étape a) sur le polymère mucoadhésif, qui peut contenir des quantités éventuelles d'autres auxiliaires pharmaceutiques usuels, et où les constituants sont présents sous forme de poudre micronisée, par agglomération rotative, extrusion ou granulation.
